# EUROPEAN PATENT APPLICATION

(11) **EP 3 978 501 A1**
(43) Date of publication of application: **06.04.2022**
(21) Application number: 20813107.8
(22) Date of filing: 29.05.2020
(51) Int. Cl.: C07D 495/04, C07D 519/00, A61K 31/519, A61P 35/00

(54) **TETRACYCLIC COMPOUNDS AS CDC7 INHIBITORS**

(30) Priority: 30.05.2019 CN 201910464384; 06.06.2019 CN 201910491339; 18.11.2019 CN 201911128459
(71) Applicant: CHIA TAI TIANQING PHARMACEUTICAL GROUP CO., LTD., Lianyungang, Jiangsu 222062 (CN)
(72) Inventor: LI, Gang, Shanghai 200131 (CN); LU, Lun, Shanghai 200131 (CN); ZHANG, Zhibo, Shanghai 200131 (CN); HU, Lihong, Shanghai 200131 (CN); DING, Charles Z., Shanghai 200131 (CN); CHEN, Shuhui, Shanghai 200131 (CN)
(74) Representative: Müller Schupfner & Partner Patent- und Rechtsanwaltspartnerschaft mbB
(86) International application number: PCT/CN2020/093480
(87) International publication number: WO 2020/239107

(57) **Abstract**

A new class of tetracyclic compounds acting as Cdc7 inhibitors; specifically disclosed are a compound represented by formula (I), isomers thereof, or pharmaceutically acceptable salts thereof.

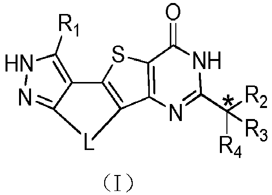

## Description

### CROSS-REFERENCE TO RELATED APPLICATIONS

The present application claims the priority to and the benefit of the Chinese Patent Application No. 201910464384.5 filed with China National Intellectual Property Administration on May 30, 2019, the Chinese Patent Application No. 201910491339.9 filed with China National Intellectual Property Administration on Jun. 6, 2019 and the Chinese Patent Application No. 201911128459.9 filed with China National Intellectual Property Administration on Nov. 18, 2019, the disclosure of each of which is incorporated herein by reference in its entirety.

### TECHNICAL FIELD

The present application relates to a new type of tetracyclic compounds as Cdc7 inhibitors, and specifically discloses a compound of formula (I), an isomer thereof or a pharmaceutically acceptable salt thereof.

### BACKGROUND

Cdc7 is a serine/threonine kinase that was first discovered in *Saccharomyces cerevisiae* in 1974, after which scientists also discovered homologous proteins to it in other eukaryotes. Different species of Cdc7 have certain differences in structure but have very similar functions. In one aspect, it activates MCM to promote the formation of replication origin complexes by phosphorylating minichromosome maintenance proteins (MCM proteins), an important element of DNA replication initiator, and in another aspect, it can also be used as an important regulatory factor of the S-phase checkpoint of a cell cycle for controlling the smooth progress of the cell cycle.

HuCdc7, a homologous protein in human cells to Cdc7, was discovered by scientists only in late 1990s. HuCdc7 is expressed in almost all histiocytes of humans. However, it is found that the abnormal high expression of huCdc7 occurs in various tumor cells of humans, and such abnormal high expression shows high correlation with abnormal proliferation and metastasis of tumors and resistance to chemotherapeutic drugs. Therefore, huCdc7 has become an important marker and target in the current tumor research.

HuCdc7 is expressed at constant levels in a normal cell cycle and is regulated by several factors and auxiliary proteins in the cell cycle and is therefore in a state of dynamic equilibrium. HuCdc7 is abnormally expressed and over-activated in tumor cells due to disturbances of the cell cycle. Hess *et al.* found that due to over-expression of huCdc7 in various tumor cells, the over-expressed huCdc7 may promote over-activation of MCM2, an important marker for tumor cells, and thus the abnormal proliferation of tumor cells. Besides, they also found that huCdc7 shows high expression in all metastatic tumor cells, suggesting that the abnormal high expression of huCdc7 may be closely associated with the metastasis of tumor cells. Nambiar *et al.* have recently found that the auxiliary protein ASK of huCdc7 is also highly expressed in multiple cutaneous melanoma cell lines, which further enhances the activity of huCdc7 in tumor cells. In addition, the abnormal high expression and activation of huCdc7 play a key role in resistance to chemotherapeutic drugs for tumor cells. Tenca *et al.* found that huCdc7 is extensively expressed with high activity after treating tumor cells with chemotherapeutic drugs Hu and etoposide, and it was noted in the research that huCdc7 inhibits the activity of the two drugs and thus reduces the damage to tumor cells by phosphorylating multiple amino acid sites of MCM2 and MCM4.

TAK-931 is a Cdc7 inhibitor and is in phase II clinical trials at present. Therefore, there is a clinical need for developing a new generation of Cdc7 inhibitor capable of being stably metabolized.

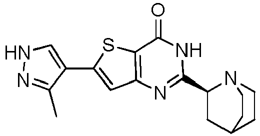

### TAK-931

### SUMMARY

The present application provides a compound of formula (I), an isomer thereof or a pharmaceutically acceptable salt thereof, 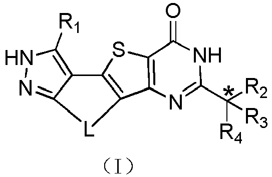 wherein,
the carbon atom with "^{∗}" can be a chiral carbon atom present in a form of a single (R) or (*S*) enantiomer or in a form rich in one enantiomer;
L is selected from the group consisting of -CH₂-CH₂-CH₂-, -CH₂-O-CH₂-, -CH₂-S-CH₂-, -CH₂-NH-CH₂-, -NH-CH₂-CH₂-, -S-CH₂-CH₂- and -O-CH₂-CH₂-;
R₁ is selected from the group consisting of H, halogen, CN, C₁₋₆ alkyl, C₃₋₆ cycloalkyl, phenyl, and 5-6 membered heteroaryl, wherein the C₁₋₆ alkyl, C₃₋₆ cycloalkyl, phenyl, and 5-6 membered heteroaryl are each independently optionally substituted with 1, 2 or 3 Rₐ, the 5-6 membered heteroaryl containing 1, 2 or 3 heteroatoms or heteroatom groups each independently selected from the group consisting of O, S, N and NH; R₂ is selected from R_{b}, R₃ is selected from NH₂, and R₄ is selected from H;
alternatively, R₂ is selected from R_{c}, and R₃ and R₄ are joined to form a ring A group optionally substituted with 1, 2 or 3 Rₑ, wherein the ring A group is selected from the group consisting of C₆₋₁₄ aryl, 5-14 membered heteroaryl, 5-12 membered heterocycloalkenyl and 4-14 membered heterocycloalkyl each independently containing 1, 2 or 3 heteroatoms or heteroatom groups independently selected from the group consisting of O, S, N and NR_{d};
Rₐ is each independently selected from the group consisting of F, Cl, Br, I, OH, CN, NH₂, -CH₃ and 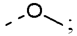
R_{b} is selected from the group consisting of H and C₁₋₆ alkyl, the C₁₋₆ alkyl being optionally substituted with 1, 2 or 3 R_{bb};
R_{c} is selected from the group consisting of H, F, Cl, Br, I and C₁₋₃ alkyl;
R_{d} is selected from the group consisting of H and C₁₋₄ alkyl;
R_{bb} is selected from the group consisting of -OCH₃, -OCH₂CH₃, -O-CH(CH₃)₂, cyclopropyl, cyclopentyl, phenyl, pyrazolyl, pyridyl, NH₂, -NHCH₃ and -N(CH₃)₂;
Rₑ is selected from the group consisting of F, Cl, Br, I, OH, CN, COOH, NH₂, -NHCH₃, -N(CH₃)₂, -CH₃, -CH₂CH₃, -CF₃, -OCH₃, -OCH₂CH₃, -O-CH(CH₃)₂, -C(=O)OCH₃, -C(=O)CH₃ and -C(=O)CH₂CH₃.

In some embodiments of the present application, the R₁ is selected from the group consisting of H, F, Cl, Br, I, CN, C₁₋₃ alkyl, C₃₋₅ cycloalkyl, phenyl and 6 membered heteroaryl, wherein the C₁₋₃ alkyl, C₃₋₅ cycloalkyl, phenyl, and 6 membered heteroaryl are each independently optionally substituted with 1, 2 or 3 Rₐ, the 6 membered heteroaryl containing 1, 2 or 3 heteroatoms selected from N.

In some embodiments of the present application, the R₁ is selected from the group consisting of H, F, Cl, Br, I, CN, -CH₃, -CH₂CH₃, cyclopropyl, phenyl and pyridyl, wherein the -CH₃, -CH₂CH₃, cyclopropyl, phenyl and pyridyl are each independently optionally substituted with 1, 2 or 3 Rₐ.

In some embodiments of the present application, the R₁ is selected from the group consisting of H, F, Cl, Br, I, CN, -CH₃, -CH₂CH₃, -CF₃, cyclopropyl, phenyl and pyridyl.

In some embodiments of the present application, the R₁ is selected from H.

In some embodiments of the present application, the Rₐ is selected from F.

In some embodiments of the present application, the R_{b} is selected from the group consisting of H and C₁₋₄ alkyl, the C₁₋₄ alkyl being optionally substituted with 1, 2 or 3 R_{bb}.

In some embodiments of the present application, the R_{b} is selected from the group consisting of H, methyl, ethyl, isopropyl, *n*-propyl, *n*-butyl and isobutyl.

In some embodiments of the present application, the R_{b} is selected from C₁₋₃ alkyl.

In some embodiments of the present application, the R_{b} is selected from isopropyl.

In some embodiments of the present application, the R_{c} is selected from the group consisting of H, F and C₁₋₃ alkyl.

In some embodiments of the present application, the R_{c} is selected from the group consisting of H, methyl, ethyl and F.

In some embodiments of the present application, the R_{c} is selected from the group consisting of H and methyl. In some embodiments of the present application, the R_{d} is selected from the group consisting of H, methyl, ethyl, *n*-propyl, isopropyl and *n*-butyl.

In some embodiments of the present application, the R_{d} is selected from the group consisting of H and C₁₋₃ alkyl.

In some embodiments of the present application, the R_{d} is selected from the group consisting of H, methyl and isopropyl.

In some embodiments of the present application, the ring A group is selected from the group consisting of C₆₋₁₀ aryl, 5-9 membered heteroaryl, 5-7 membered heterocycloalkenyl and 4-10 membered heterocycloalkyl optionally substituted with 1, 2 or 3 Rₑ and each independently containing 1, 2 or 3 heteroatoms or heteroatom groups independently selected from the group consisting of O, S, N and NR_{d}.

In some embodiments of the present application, the ring A group is selected from 5-9 membered heterocycloalkyl optionally substituted with 1, 2 or 3 Rₑ, the 5-9 membered heterocycloalkyl containing 1, 2 or 3 heteroatoms or heteroatom groups independently selected from the group consisting of O, S, N and NR_{d}.

In some embodiments of the present application, the ring A group is selected from the group consisting of 5 membered, 6 membered, 7 membered and 8 membered heterocycloalkyl optionally substituted with 1, 2 or 3 Rₑ, the 5 membered, 6 membered, 7 membered and 8 membered heterocycloalkyl containing 1, 2 or 3 heteroatoms or heteroatom groups independently selected from the group consisting of O, S, N and NR_{d}.

In some embodiments of the present application, the ring A group is selected from 5-9 membered heterocycloalkyl optionally substituted with 1, 2 or 3 Rₑ and containing 1 heteroatom or heteroatom group selected from the group consisting of N and NR_{d}.

In some embodiments of the present application, the ring A group is selected from the group consisting of 5 membered, 6 membered, 7 membered and 8 membered heterocycloalkyl optionally substituted with 1, 2 or 3 Rₑ, the 5 membered, 6 membered, 7 membered and 8 membered heterocycloalkyl containing 1 heteroatom or heteroatom group selected from the group consisting of N and NR_{d}.

In some embodiments of the present application, the ring A group is selected from 5-9 membered heterocycloalkyl containing 1, 2 or 3 heteroatoms or heteroatom groups independently selected from the group consisting of N and NR_{d}.

In some embodiments of the present application, the ring A group is selected from 5-9 membered heterocycloalkyl containing 1 heteroatom or heteroatom group selected from the group consisting of N and NR_{d}.

In some embodiments of the present application, the ring A group is selected from the group consisting of 5 membered, 6 membered, 7 membered and 8 membered heterocycloalkyl containing 1 heteroatom or heteroatom group selected from the group consisting of N and NR_{d}.

In some embodiments of the present application, the ring A group is selected from the group consisting of pyrrolidinyl, piperidinyl, morpholinyl, 1-azabicyclo[2.2.2]octyl, 1-azabicyclo[2.2.1]heptanyl, 1-azabicyclo[3.2.2]nonyl and azepanyl optionally substituted with 1, 2 or 3 Rₑ, and contains 1 heteroatom or heteroatom group selected from the group consisting of N and NR_{d}.

In some embodiments of the present application, the ring A group is selected from the group consisting of pyrrolidinyl, piperidinyl, morpholinyl, 1-azabicyclo[2.2.2]octanyl, 1-azabicyclo[2.2.1]heptanyl, 1-azabicyclo[3.2.2]nonanyl and azepanyl, and contains 1 heteroatom or heteroatom group selected from the group consisting of N and NR_{d}. In some embodiments of the present application, the ring A group is selected from the group consisting of 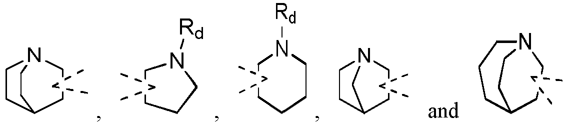 optionally substituted with 1, 2 or 3 Rₑ.

In some embodiments of the present application, the ring A group is selected from the group consisting of

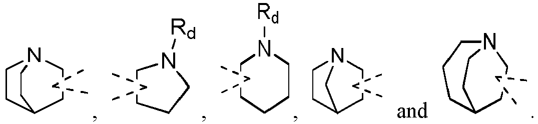

In some embodiments of the present application, the structural unit 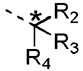 is selected from 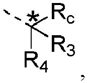 further from the group consisting of

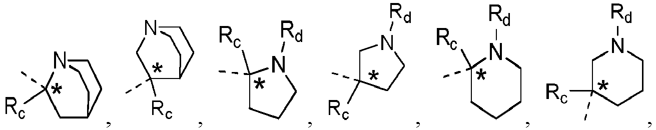

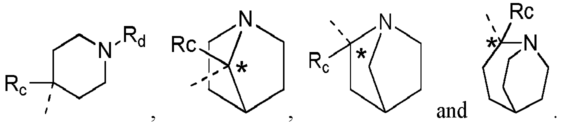

In some embodiments of the present application, the structural unit 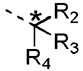 is selected from 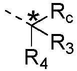 , the carbon atom to which R₃, R₄ and R_{c} are collectively connected being a chiral carbon atom; further from the group consisting of 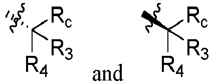 ; further more from the group consisting of 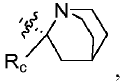 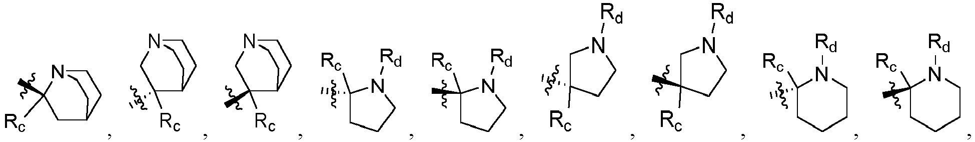 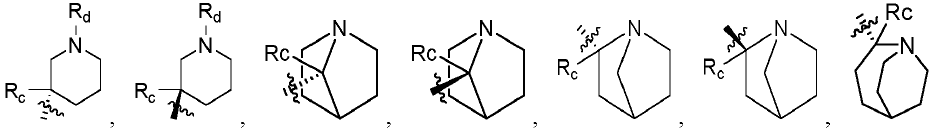 and

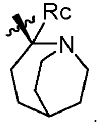

In some embodiments of the present application, the structural unit 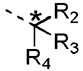 is selected from 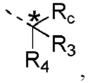 further from the group consisting of

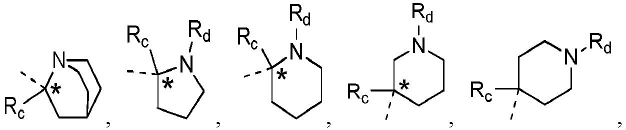

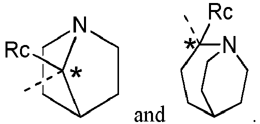

In some embodiments of the present application, the structural unit 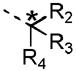 is selected from 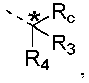 the carbon atom to which R₃, R₄ and R_{c} are collectively connected being a chiral carbon atom; further from the group consisting of 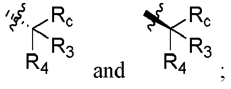 further more from the group consisting of

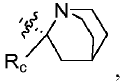

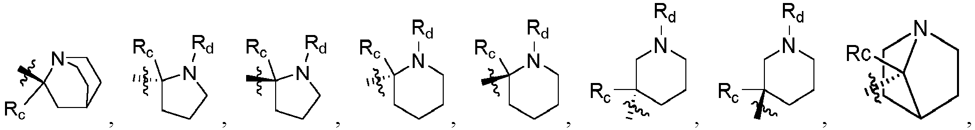

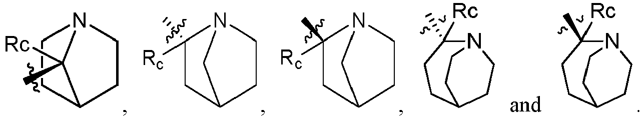

In some embodiments of the present application, the structural unit 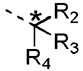 is selected from 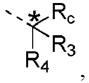 further from the group consisting of

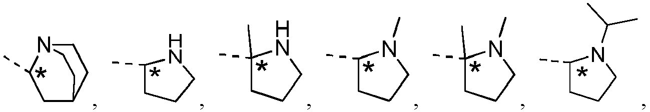

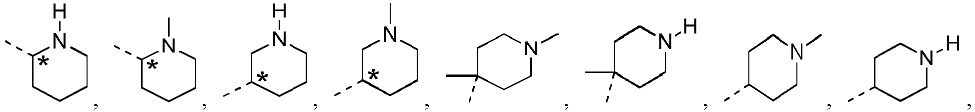

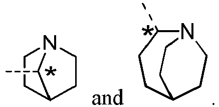

In some embodiments of the present application, the structural unit 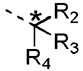 is selected from 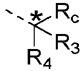 , the carbon atom to which R₃, R₄ and R_{c} are collectively connected being a chiral carbon atom; further from the group consisting of 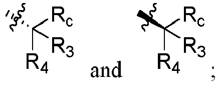 further more from the group consisting of

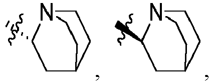

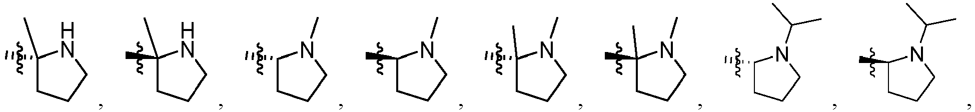

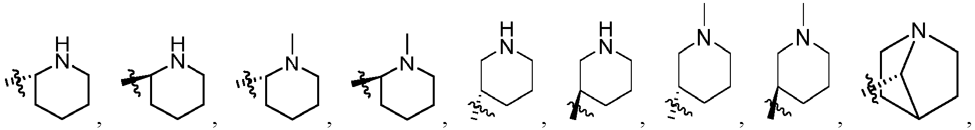

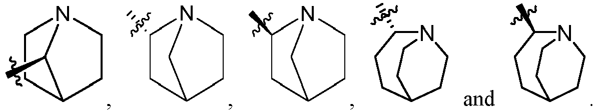

In some embodiments of the present application, the structural unit 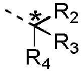 is selected from 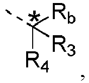 further from 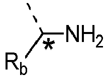 , further more from

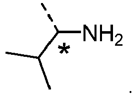

In some embodiments of the present application, the structural unit 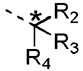 is selected from 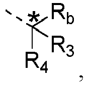 the carbon atom to which R₃, R₄ and R_{b} are collectively connected being a chiral carbon atom; further from the group consisting of 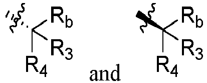 ; further more from the group consisting of 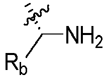 and 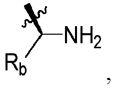 still further from the group consisting of

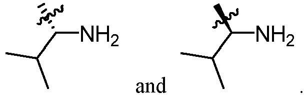

In some embodiments of the present application, the L is selected from the group consisting of -CH₂-CH₂-CH₂-, -CH₂-O-CH₂-, -CH₂-S-CH₂- and -CH₂-NH-CH₂-.

In some embodiments of the present application, the L is selected from -CH₂-CH₂-CH₂-.

In some embodiments of the present application, the R₁ is selected from the group consisting of H, F, Cl, Br, I, CN, -CH₃, -CH₂CH₃, cyclopropyl, phenyl and pyridyl, wherein the -CH₃, -CH₂CH₃, cyclopropyl, phenyl and pyridyl are each independently optionally substituted with 1, 2 or 3 Rₐ, and the other variables are as defined herein.

In some embodiments of the present application, the R₁ is selected from the group consisting of H, F, Cl, Br, I, CN, -CH₃, -CH₂CH₃, -CF₃, cyclopropyl, phenyl and pyridyl, and the other variables are as defined herein.

In some embodiments of the present application, the R₁ is selected from H, and the other variables are as defined herein.

In some embodiments of the present application, the R_{b} is selected from the group consisting of H, methyl, ethyl, isopropyl, *n*-propyl, *n*-butyl and isobutyl, and the other variables are as defined herein.

In some embodiments of the present application, the R_{b} is selected from isopropyl, and the other variables are as defined herein.

In some embodiments of the present application, the R_{c} is selected from the group consisting of H, methyl, ethyl and F, and the other variables are as defined herein.

In some embodiments of the present application, the R_{c} is selected from the group consisting of H and methyl, and the other variables are as defined herein.

In some embodiments of the present application, the R_{d} is selected from the group consisting of H, methyl, ethyl, propyl, isopropyl and *n*-butyl, and the other variables are as defined herein.

In some embodiments of the present application, the R_{d} is selected from the group consisting of H, methyl and isopropyl, and the other variables are as defined herein.

In some embodiments of the present application, the ring A group is selected from 5-9 membered heterocycloalkyl containing 1 heteroatom or heteroatom group selected from the group consisting of N and NR_{d}, and the other variables are as defined herein.

In some embodiments of the present application, the ring A group is selected from the group consisting of 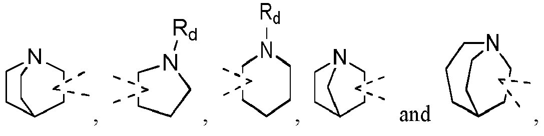 and the other variables are as defined herein.

In some embodiments of the present application, the structural unit 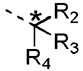 is selected from 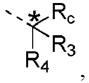 further from the group consisting of 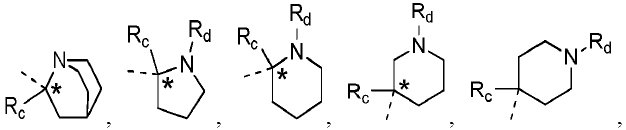 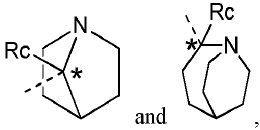 and the other variables are as defined herein.

In some embodiments of the present application, the structural unit 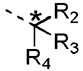 is selected from 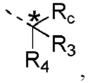 further from the group consisting of 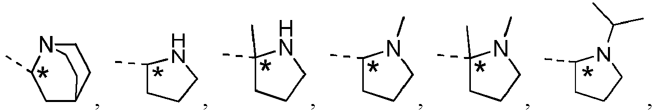 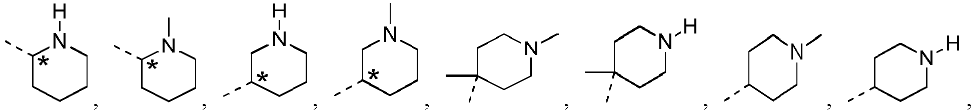 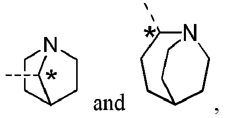 and the other variables are as defined herein.

In some embodiments of the present application, the structural unit 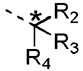 is selected from 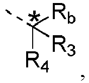 further from 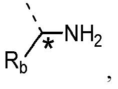 and the other variables are as defined herein.

In some embodiments of the present application, the structural unit 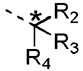 is selected from 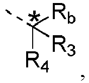 further from 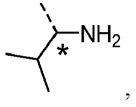 and the other variables are as defined herein.

In some embodiments of the present application, the L is selected from -CH₂-CH₂-CH₂-, and the other variables are as defined herein.

In some embodiments of the present application, provided are the compound of formula (I), the isomer thereof or the pharmaceutically acceptable salt thereof, wherein,
the carbon atom with "^{∗}" can be a chiral carbon atom present in a form of a single (*R*) or (*S*) enantiomer or in a form rich in one enantiomer;
L is selected from -CH₂-CH₂-CH₂-;
R₁ is selected from H;
R₂ is selected from R_{b}, R₃ is selected from NH₂, and R₄ is selected from H;
alternatively, R₂ is selected from R_{c}, and R₃ and R₄ are joined to form a ring A group selected from 4-14 membered heterocycloalkyl each independently containing 1, 2 or 3 heteroatoms or heteroatom groups independently selected from the group consisting of N and NR_{d};
R_{b} is selected from C₁₋₆ alkyl;
R_{c} is selected from the group consisting of H and C₁₋₃ alkyl;
R_{d} is selected from the group consisting of H and C₁₋₄ alkyl.

In some embodiments of the present application, the compound is selected from the group consisting of a compound of formula (I-1) and a compound of formula (1-2), an isomer thereof or a pharmaceutically acceptable salt thereof, 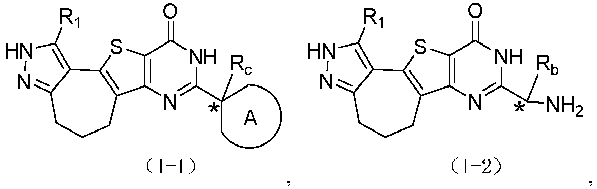 wherein,
the carbon atom with "^{∗}" can be a chiral carbon atom present in a form of a single (*R*) or (*S*) enantiomer or in a form rich in one enantiomer; R₁ and R_{c} are as defined in the compound of formula (I) disclosed herein; the ring A group is as defined in the compound of formula (I) disclosed herein; R_{b} is as defined for the compound of formula (I) disclosed herein.

In some embodiments of the present application, the structural unit 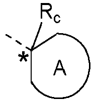 is defined as the structural unit 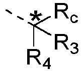 is.

In some embodiments of the present application, the compound is selected from the group consisting of a compound of formula (I-1a), a compound of formula (I-1b), a compound of formula (I-2a) and a compound of formula (I-2b), an isomer thereof or a pharmaceutically acceptable salt thereof, 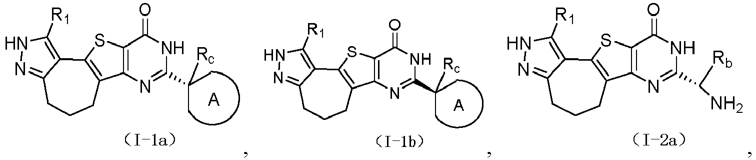 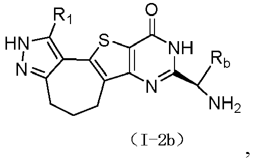 wherein,
R₁ and R_{c} are as defined in the compound of formula (I) disclosed herein, and the carbon atom to which R_{c} and the ring A group are collectively connected is a chiral carbon atom; the ring A group is as defined in the compound of formula (I) disclosed herein; R_{b} is as defined for the compound of formula (I) disclosed herein.

In some embodiments of the present application, the structural unit 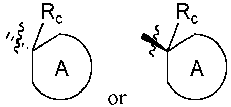 is defined as the structural unit 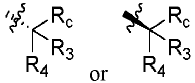 is, respectively.

In some embodiments of the present application, the compound is selected from the group consisting of a compound of formula (I-11), a compound of formula (1-12), a compound of formula (1-13), a compound of formula (1-14), a compound of formula (1-15) and a compound of formula (1-16), an isomer thereof or a pharmaceutically acceptable salt thereof, 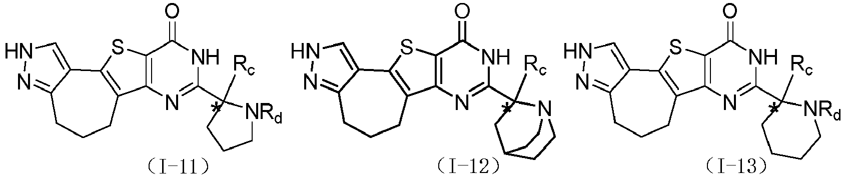 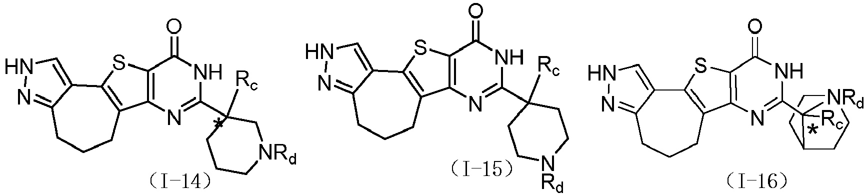 wherein,
the carbon atom with "^{∗}" is a chiral carbon atom present in a form of a single (*R*) or (*S*) enantiomer or in a form rich in one enantiomer; R₁, R_{c} and R_{d} are as defined for the compound of formula (I) disclosed herein.

In some embodiments of the present application, the compound is selected from the group consisting of a compound of formula (I-11a), a compound of formula (I-11b), a compound of formula (I-12a), a compound of formula (I-12b), a compound of formula (I-13a), a compound of formula (I-13b), a compound of formula (I-14a), a compound of formula (I-14b), a compound of formula (I-16a) and a compound of formula (I-16b), an isomer thereof or a pharmaceutically acceptable salt thereof, 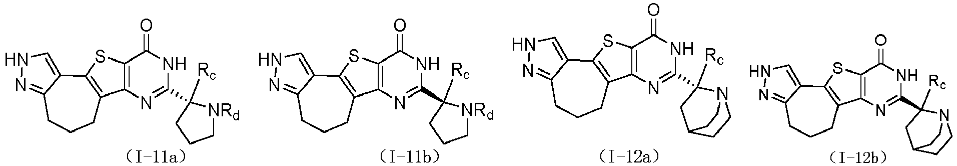 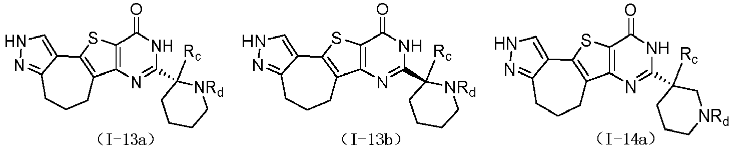 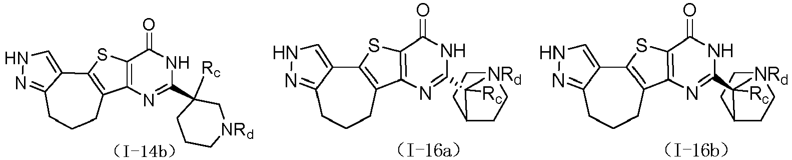 wherein,
R₁, R_{c} and R_{d} are as defined for the compound of formula (I) disclosed herein.

In some aspects, the present application encompasses the variables defined above and solutions thereof, as well as any combination thereof.

The present application also provides a compound of the formula below, an isomer thereof or a pharmaceutically acceptable salt thereof,

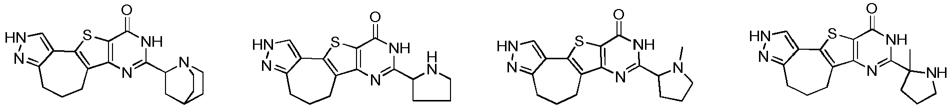

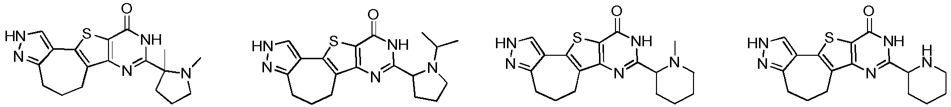

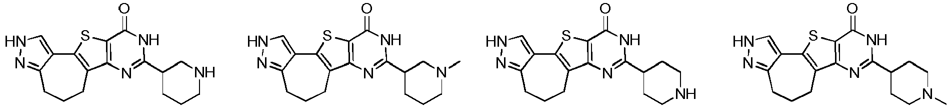

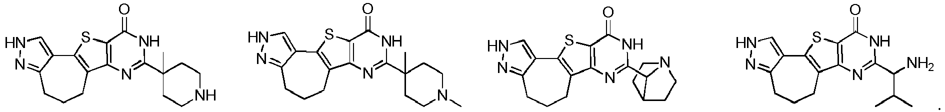

In some embodiments of the present application, provided are the compound, the isomer thereof or the pharmaceutically acceptable salt thereof, selected from the group consisting of

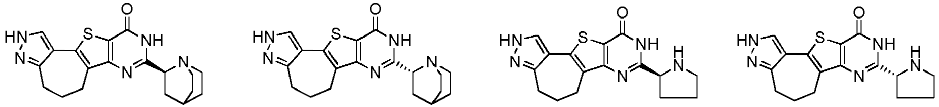

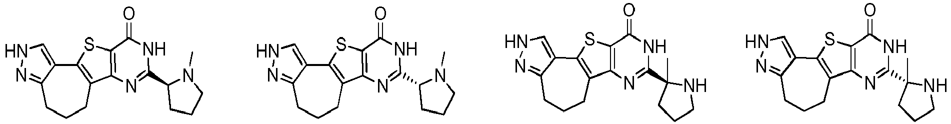

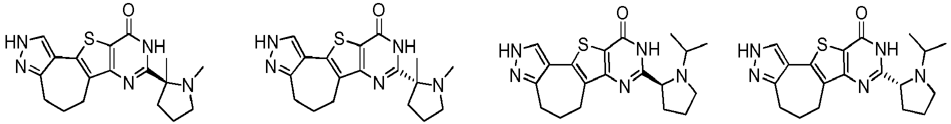

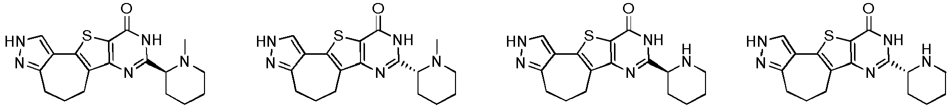

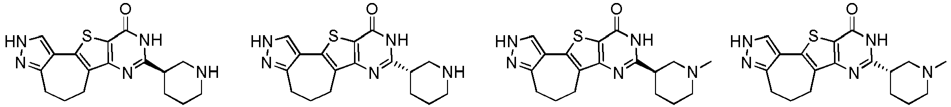

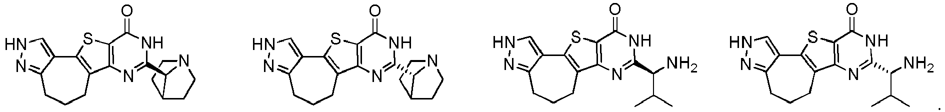

The present application further provides a pharmaceutical composition comprising a therapeutically effective amount of the compound, the isomer thereof or the pharmaceutically acceptable salt thereof and a pharmaceutically acceptable carrier.

The present application also provides use of the compound, the isomer thereof or the pharmaceutically acceptable salt thereof or the pharmaceutical composition in preparing a therapeutic Cdc7 inhibitor.

The present application also provides use of the compound, the isomer thereof or the pharmaceutically acceptable salt thereof or the pharmaceutical composition in preparing a medicament for treating a tumor.

The present application also provides a method for treating a Cdc7 kinase-mediated disease, comprising administering to a mammal, preferably a human, in need of such treatment a therapeutically effective amount of the compound, the isomer thereof or the pharmaceutically acceptable salt thereof or the pharmaceutical composition.

The present application also provides use of the compound, the isomer thereof or the pharmaceutically acceptable salt thereof or the pharmaceutical composition in treating a Cdc7 kinase-mediated disease.

The present application also provides the compound, the isomer thereof or the pharmaceutically acceptable salt thereof or the pharmaceutical composition for use in treating a Cdc7 kinase-mediated disease.

The present application also provides the compound, the isomer thereof or the pharmaceutically acceptable salt thereof for use as a medicament. In some embodiments of the present application, the use as a medicament refers to use as a medicament for treating a Cdc7 kinase-mediated disease.

In some embodiments of the present application, the Cdc7 inhibitor is a medicament for treating a tumor.

In some embodiments of the present application, the tumor includes colorectal cancer and pancreatic cancer.

In some embodiments of the present application, the medicament for treating a tumor refers to a medicament for treating colorectal cancer and pancreatic cancer.

### Technical Effects

As a Cdc7 inhibitor, the compound disclosed herein has a wide application prospect in treating tumors. The compound disclosed herein has strong inhibitory activity against Cdc7/DBF4, and also shows good inhibitory activity against Colo205 cells. In addition, the compound disclosed herein has good AUC₀₋ₗₐₛₜ and bioavailability and significant inhibitory effects on tumors in mice. Therefore, further intensive research on the Cdc7 kinase and inhibitors thereof is expected to pave a new way for clinically treating tumors. The compound disclosed herein is expected to become a new medicament with better therapeutic effects and lower toxic and side effects compared to similar products.

### Definitions and Description

Unless otherwise stated, the following terms and phrases used herein are intended to have the following meanings. A particular term or phrase, unless otherwise specifically defined, should not be considered as uncertain or unclear, but construed according to its common meaning. When referring to a trade name, it is intended to refer to its corresponding commercial product or its active ingredient.

The term "pharmaceutically acceptable" is used herein for those compounds, materials, compositions and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of the compound disclosed herein, which is prepared from the compound having particular substituents disclosed herein and a relatively nontoxic acid or base. When the compound disclosed herein contains a relatively acidic functional group, a base addition salt can be obtained by contacting such a compound with a sufficient amount of a base in a pure solution or a suitable inert solvent. Pharmaceutically acceptable base addition salts include sodium, potassium, calcium, ammonium, organic amine, or magnesium salts, or similar salts. When the compound disclosed herein contains a relatively basic functional group, an acid addition salt can be obtained by contacting such a compound with a sufficient amount of an acid in a pure solution or a suitable inert solvent. Examples of pharmaceutically acceptable acid addition salts include salts derived from inorganic acids, such as hydrochloric acid, hydrobromic acid, nitric acid, carbonic acid, bicarbonate radical, phosphoric acid, monohydrogen phosphate, dihydrogen phosphate, sulfuric acid, hydrogen sulfate, hydroiodic acid and phosphorous acid; and salts derived from organic acids, such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid and methanesulfonic acid. Also included are salts of amino acids (e.g., arginine) and salts of organic acids such as glucuronic acid. Some particular compounds disclosed herein contain both basic and acidic groups and thus can be converted to any base or acid addition salts.

The pharmaceutically acceptable salts disclosed herein can be synthesized from a parent compound having an acidic or basic group by conventional chemical methods. In general, such salts are prepared by the following method: the free acid or base form of the compound reacting with a stoichiometric amount of the appropriate base or acid in water or an organic solvent or a mixture thereof.

The pharmaceutically acceptable salt disclosed herein can be converted into the free state by using a known method or a method similar thereto, for example, by reacting a pharmaceutically acceptable acid addition salt or base addition salt with a stoichiometric amount of an appropriate base or acid.

The compounds disclosed herein can be in the form of a geometric isomer or stereoisomer. All such compounds are contemplated herein, including *cis* and *trans* isomers, (-)- and (+)- enantiomers, (R)- and (S)-enantiomers, diastereoisomers, (*D*)-isomers, (*L*)-isomers, and racemic mixtures and other mixtures thereof, such as an enantiomer or diastereoisomer enriched mixture, all of which are encompassed within the scope of the present application. Substituents such as alkyl may have an additional asymmetric carbon atom. All these isomers and mixtures thereof are encompassed within the scope of the present application.

The carbon atom with "^{∗}" disclosed herein may be a chiral carbon atom, which means either a chiral carbon atom or an achiral carbon atom, depending on the connection of the carbon atom in the structure of a compound.

Unless otherwise stated, the term "enantiomer" or "optical isomer" refers to stereoisomers that are mirror images of each other.

Unless otherwise stated, the term *"cis-trans* isomer" or "geometric isomer" results from the inability of a single bond of a ring carbon atom or a double bond to rotate freely.

Unless otherwise stated, the term "diastereomer" refers to stereoisomers whose molecules have two or more chiral centers and are not mirror images of each other.

Unless otherwise stated, "(+)" stands for dextrorotation, "(-)" stands for levorotation, and "(±)" stands for racemization.

Unless otherwise stated, the absolute configuration of a stereogenic center is represented by a wedged solid bond ( 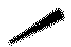 ) and a wedged dashed bond ( 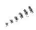 ), and the relative configuration of a stereogenic center is represented by a straight solid bond ( 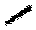 ) and a straight dashed bond ( 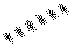 ). A wavy line ( 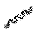 ) represents a wedged solid bond ( 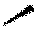 ) or a wedged dashed bond ( 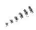 ), or a wavy line ( 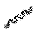 ) represents a straight solid bond ( 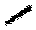 ) or a straight dashed bond ( 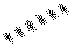 ).

Unless otherwise stated, the term "rich in one isomer", "isomer-rich", "rich in one enantiomer", or "enantiomer-rich" means that the content of one of the isomers or enantiomers is less than 100% and more than or equal to 60%, 70%, 80%, 90%, 95%, 96%, 97%, 98%, 99%, 99.5%, 99.6%, 99.7%, 99.8% or 99.9%. Optically active (R)- and (S)-isomers and *D* and *L* isomers can be prepared by chiral synthesis or chiral reagents or other conventional techniques. An enantiomer of certain compound disclosed herein can be prepared by asymmetric synthesis or derivatization using a chiral auxiliary, wherein the resulting diastereoisomeric mixture is separated and the auxiliary group is cleaved so as to provide the desired pure enantiomer. Alternatively, when the molecule contains a basic functional group (such as amino) or an acidic functional group (such as carboxyl), the compound reacts with an appropriate optically active acid or base to form a salt of the diastereoisomer, which is then subjected to diastereoisomeric resolution through conventional methods in the art to give the pure enantiomer. Furthermore, the enantiomer and the diastereoisomer are generally isolated through chromatography using a chiral stationary phase, optionally in combination with chemical derivatization (e.g., carbamate generated from amines). The compound disclosed herein may contain an unnatural proportion of atomic isotope at one or more of the atoms that constitute the compound. For example, the compound may be labeled with a radioisotope, such as tritium (³H), iodine-125 (¹²⁵I), or C-14 (¹⁴C). For another example, hydrogen can be substituted with deuterium to form a deuterated drug, and the bond formed by deuterium and carbon is firmer than that formed by common hydrogen and carbon. Compared with an un-deuterated drug, the deuterated drug has the advantages of reduced toxic side effect, increased stability, enhanced efficacy, prolonged biological half-life and the like. All isotopic variations of the compound described herein, whether radioactive or not, are encompassed within the scope of the present application.

Unless otherwise specified, the compounds disclosed herein include both E and Z geometric isomers when they contain olefinic double bonds or other centers of geometric asymmetry. Likewise, all tautomeric forms are encompassed within the scope of the present application. For example, 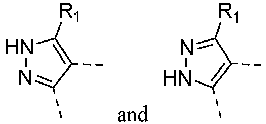 are tautomeric to each other; for another example, 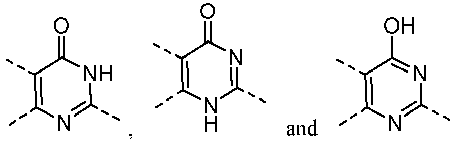 are tautomeric to one another.

The term "substituted" means that one or more hydrogen atoms on a specific atom are substituted with substituents which may include deuterium and hydrogen variants, as long as the valence of the specific atom is normal and the substituted compound is stable. When the substituent is an oxygen (i.e., =O), it means that two hydrogen atoms are substituted. Substitution of oxygen does not occur on aromatic groups. The term "optionally substituted" means that an atom can be substituted with a substituent or not. Unless otherwise specified, the type and number of the substituent may be arbitrary as long as being chemically achievable. When any variable (e.g., R) occurs more than once in the constitution or structure of a compound, the variable is independently defined in each case. Thus, for example, if a group is substituted with 0-2 R, the group can be optionally substituted with two R at most, and the definition of R in each case is independent. Furthermore, a combination of a substituent and/or a variant thereof is permissible only if the combination can result in a stable compound.

When a bond of a substituent can be connected to one or more atoms on a ring, the substituent can be bonded to any atom on the ring. For example, the structural unit 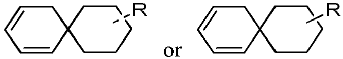 represents that the substitution of substituent R may occur in any one position on cyclohexyl or cyclohexadienyl. When it is not specified by which atom the listed substituent is connected to the group to be substituted, the substituent can be connected via any atom of the group. For example, pyridinyl as a substituent can be connected to the group to be substituted through any carbon atom on the pyridine ring.

When bonds of two substituents can be connected to the same carbon atom on a ring, the substituents can be bonded to any one of the same carbon atoms on the ring. For example, the structural unit 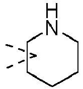 represents that the substitution of two substituents may occur at any one of the same carbon atoms of the piperidine ring, and thus, the structural unit 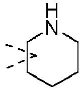 includes 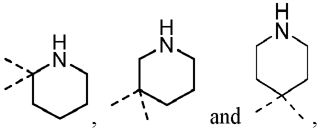 but not structural units such as 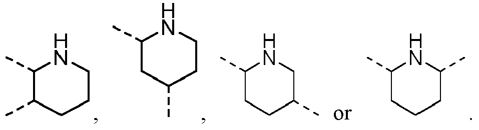 When the enumerative linking group does not indicate the direction for linking, the direction for linking is arbitrary. For example, when the linking group L contained in 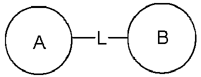 is -M-W-, -M-W- can either link ring A and ring B in a direction same as left-to-right reading order to form 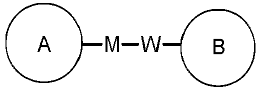 , or link ring A and ring B in an opposing direction to form 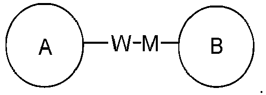 A combination of the linking group, a substituent and/or a variant thereof is permissible only if the combination can result in a stable compound.

Unless otherwise specified, when a group has one or more connectable sites, any one or more of the sites of the group may be connected to other groups by chemical bonds. When there is no designated connecting mode for a chemical bond and H atoms are present at a connectable site, the number of the H atoms at the connectable site is correspondingly reduced based on the number of the connected chemical bonds, and a group with a corresponding valence number is thus formed. The chemical bond that connects the site to another group may be represented by a straight solid bond ( 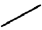 ), a straight dashed bond ( 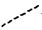 ), or a wavy line ( 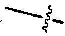 ). For example, the straight solid bond in -OCH₃ refers to being connected to another group via the oxygen atom in the group; the straight dashed bond in 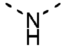 refers to being connected to another group via two ends of the nitrogen atom in the group; the wavy line in 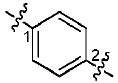 refers to being connected to another group via the carbon atoms at positions 1 and 2 in the phenyl group; 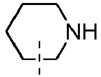 means that any connectable site on the piperidinyl can be connected to another group via 1 bond, and at least 4 connecting modes 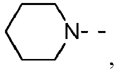 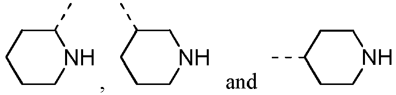 are possible; even if -N- is connected to an H atom, 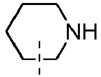 includes the connecting mode of 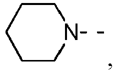 except that when 1 bond is connected to a site, the number of H at that site is correspondingly reduced by 1 and a monovalent piperidinyl is thus formed.

Unless otherwise specified, the number of atoms on a ring is generally defined as the member number of the ring. For example, "5-7 membered ring" refers to a "ring" on which 5 to 7 atoms are arranged in a circle.

Unless otherwise specified, the term "C₁₋₆ alkyl" refers to a linear or branched saturated hydrocarbon group consisting of 1 to 6 carbon atoms. The C₁₋₆ alkyl includes C₁₋₅, C₁₋₄, C₁₋₃, C₁₋₂, C₂₋₆, C₂₋₄, C₆, and C₅ alkyl, etc., and may be monovalent (e.g., methyl), divalent (e.g., methylene), or polyvalent (e.g., methenyl). Examples of C₁₋₆ alkyl include, but are not limited to, methyl (Me), ethyl (Et), propyl (including *n*-propyl and isopropyl), butyl (including *n*-butyl, isobutyl, *s*-butyl, and *t*-butyl), pentyl (including *n*-pentyl, isopentyl, and neopentyl), hexyl, and the like.

Unless otherwise specified, the term "halogen", by itself or as part of another substituent, refers to a fluorine, chlorine, bromine or iodine atom.

Unless otherwise specified, "C₃₋₆ cycloalkyl" refers to a saturated cyclic hydrocarbon group consisting of 3 to 6 carbon atoms, which is a monocyclic and bicyclic ring system, wherein the carbon atoms may optionally be oxidized (i.e., C=O). The C₃₋₆ cycloalkyl includes C₃₋₅, C₄₋₅, C₅₋₆ cycloalkyl and the like, and may be monovalent, divalent or polyvalent. Examples of C₃₋₆ cycloalkyl include, but are not limited to, cyclopropyl, cyclobutyl, cyclopentyl, cyclohexyl and the like.

Unless otherwise specified, the term "4-14 membered heterocycloalkyl", by itself or in combination with other terms, refers to a saturated cyclic group consisting of 4 to 14 ring atoms, of which 1, 2, 3 or 4 ring atoms are heteroatoms independently selected from the group consisting of O, S, and N, with the remaining being carbon atoms. The nitrogen atom is optionally quaternized, and the carbon, nitrogen and sulfur heteroatoms can be optionally oxidized (i.e., C=O, NO and S(O)ₚ, wherein p is 1 or 2). This includes monocyclic, bicyclic and tricyclic systems, wherein the bicyclic and tricyclic systems include spirocyclic, fused and bridged rings. Furthermore, with respect to the "4-14 membered heterocycloalkyl", a heteroatom may occupy the position where the heterocycloalkyl is connected to the rest of the molecule. The 4-14 membered heterocycloalkyl includes 4-12 membered, 4-10 membered, 5-10 membered, 5-9 membered, 5-8 membered, 3-10 membered, 3-8 membered, 3-6 membered, 3-5 membered, 4-6 membered, 5-6 membered, 4 membered, 5 membered and 6 membered heterocycloalkyl and the like. Examples of 4-14 membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl, dioxepanyl, 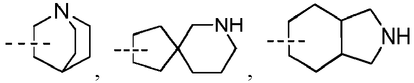 or the like.

Unless otherwise specified, the term "5-12 membered heterocycloalkenyl", by itself or in combination with other terms, refers to a partially unsaturated cyclic group consisting of 5 to 12 ring atoms containing at least one carbon-carbon double bond, of which 1, 2, 3 or 4 ring atoms are heteroatoms independently selected from the group consisting of O, S, and N, with the remaining being carbon atoms. The nitrogen atom is optionally quaternized, and the carbon, nitrogen and sulfur heteroatoms can be optionally oxidized (i.e., C=O, NO and S(O)ₚ, wherein p is 1 or 2). This includes monocyclic, bicyclic and tricyclic systems, wherein the bicyclic and tricyclic systems include spirocyclic, fused and bridged rings, and any ring of these systems is nonaromatic. Furthermore, with respect to the "5-12 membered heterocycloalkenyl", a heteroatom may occupy the position where the heterocycloalkenyl is connected to the rest of the molecule. The 5-12 membered heterocycloalkenyl includes 5-10 membered, 5-8 membered, 5-6 membered, 4-5 membered, 4 membered, 5 membered, 6 membered heterocycloalkenyl and the like. Examples of 5-12 membered heterocycloalkenyl include, but are not limited to,

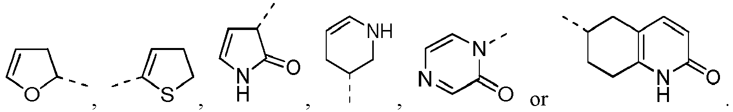

Unless otherwise specified, the term "C₆₋₁₄ aryl" herein refers to a cyclic hydrocarbon group consisting of 6 to 14 carbon atoms and having a conjugated π-electron system, which may be a monocyclic, fused bicyclic, or fused tricyclic ring system, wherein each ring is aromatic. It may be monovalent, divalent or polyvalent, and the C₆₋₁₄ aryl includes C₆₋₁₀, C₆₋₉, C₆₋₈, C₁₂, C₁₄, C₁₀ and C₆ aryl and the like. Examples of C₆₋₁₄ aryl include, but are not limited to, phenyl, naphthyl (including 1-naphthyl, 2-naphthyl, etc.) and anthryl.

Unless otherwise specified, the term "5-14 membered heteroaryl" herein refers to a cyclic group consisting of 5 to 14 ring atoms and having a conjugated π-electron system, of which 1, 2, 3 or 4 ring atoms are heteroatoms independently selected from the group consisting of O, S and N, with the remaining being carbon atoms. The nitrogen atom is optionally quaternized, and the carbon, nitrogen and sulfur heteroatoms may optionally be oxidized (i.e., C=O, NO and S(O)ₚ, wherein p is 1 or 2). It can be a monocyclic, fused bicyclic or fused tricyclic system, wherein the rings are aromatic. The 5-14 membered heteroaryl can be linked to the rest of the molecule through a heteroatom or a carbon atom. The 5-14 membered heteroaryl includes 5-12 membered, 5-10 membered, 5-8 membered, 5-7 membered, 5-6 membered, 5 membered, 6 membered heteroaryl and the like. Examples of the 5-12 membered heteroaryl include, but are not limited to, pyrrolyl (including *N*-pyrrolyl, 2-pyrrolyl, 3-pyrrolyl, etc.), pyrazolyl (including 2-pyrazolyl, 3-pyrazolyl, etc.), imidazolyl (including *N*-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (including 1*H*-1,2,3-triazolyl, 2*H*-1,2,3-triazolyl, 1*H*-1,2,4-triazolyl, 4*H*-1,2,4-triazolyl, etc.), tetrazolyl, isoxazolyl (including 3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), thienyl (including 2-thienyl, 3-thienyl, etc.), pyridinyl (including 2-pyridinyl, 3-pyridinyl, 4-pyridinyl, etc.), pyrazinyl, pyrimidinyl (including 2-pyrimidinyl, 4-pyrimidinyl, etc.), benzothiazolyl (including 5-benzothiazolyl, etc.), purinyl, benzimidazolyl (including 2-benzimidazolyl, etc.), benzoxazolyl, indolyl (including 5-indolyl, etc.), isoquinolinyl (including 1-isoquinolinyl, 5-isoquinolinyl, etc.), quinoxalinyl (including 2-quinoxalinyl, 5-quinoxalinyl, etc.), quinolyl (including 3-quinolyl, 6-quinolyl, etc.) or benzoisoquinolin.

Unless otherwise specified, the term "heteroatom or heteroatom group (i.e., heteroatom-containing atom group)" includes atoms other than carbon (C) and hydrogen (H) as well as atom groups containing these heteroatoms, for example, oxygen (O), nitrogen (N), sulfur (S), silicon (Si), germanium (Ge), aluminum (Al), boron (B), -O-, -S-, =O, =S, -C(=O)O-, -C(=O)-, -C(=S)-, -S(=O), -S(=O)₂-, and optionally substituted -C(=O)N(H)-, -N(H)-, -C(=NH)-, -S(=O)₂N(H)- or -S(=O)N(H)-.

The compounds disclosed herein can be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples disclosed herein.

An important consideration in synthesis route planning in the art is the selection of suitable protecting groups for reactive functional groups (e.g., amino in the present application). For example, reference may be made to Greene's Protective Groups in Organic Synthesis (4th Ed.) Hoboken, New Jersey: John Wiley & Sons, Inc. All references cited herein are incorporated by reference in their entirety. General synthetic route: 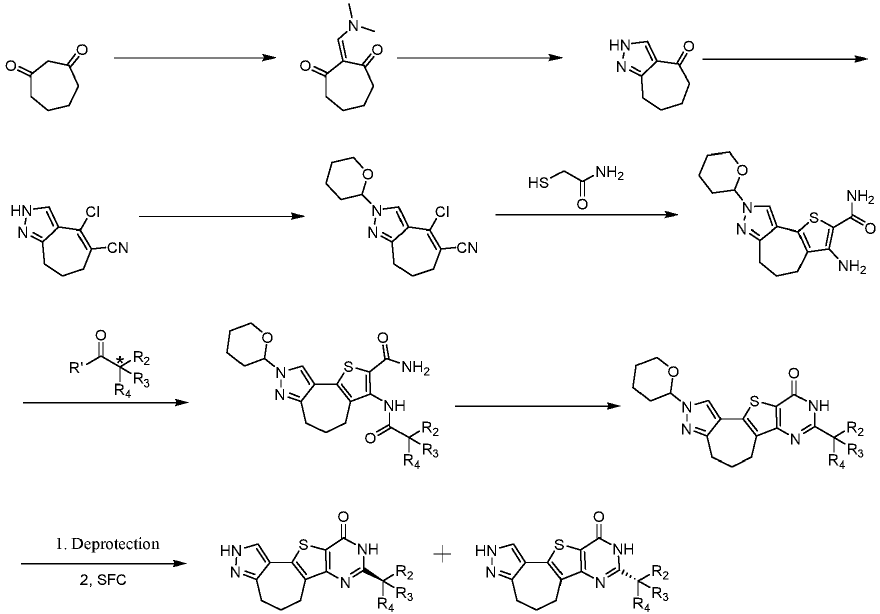 wherein,
R' is Cl or 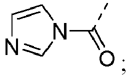
R₂, R₃ and R₄ are as described for the compound of formula (I).

The solvents used herein can be commercially available.

The following abbreviations are used herein: DMF represents *N*,*N-*dimethylformamide; DMSO represents dimethyl sulfoxide; BID represents administration twice daily.

Compounds are named according to conventional nomenclature rules in the art or using ChemDraw^{®} software, and supplier's catalog names are given for commercially available compounds.

### BRIEF DESCRIPTION OF THE DRAWINGS

FIG. 1 is a graph of tumor growth curves of tumor-bearing mice of a xenograft tumor model of the human colorectal cancer cell SW620 after administration of test compounds; and
FIG. 2 is a picture of tumors of mice in a subcutaneous xenograft tumor nude mouse model of the human colorectal cancer cell SW620.

### DETAILED DESCRIPTION

The present application is described in detail below by way of examples. However, this is by no means disadvantageously limiting the scope of the present application. Although the present application has been described in detail herein and specific examples have also been disclosed, it will be apparent to those skilled in the art that various changes and modifications can be made to the specific embodiments without departing from the spirit and scope of the present application.

### Example 1. Compound 1-1 Hydrochloride and Compound 1-2 Hydrochloride

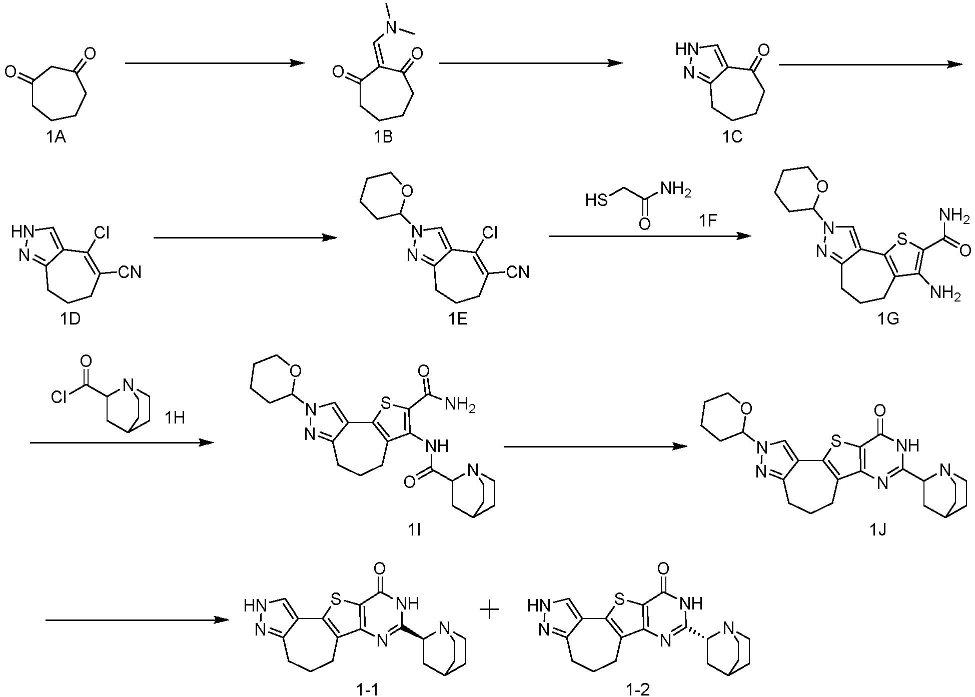

### Preparation of compound 1B:

Compound 1A (2 g) was stirred in *N*,*N*-dimethylformamide dimethyl acetal (5.67 g) at 100 °C for 3 h. After the reaction was completed, the reaction mixture was concentrated to dryness under reduced pressure to give compound 1B for direct use in the next step without purification. LCMS (ESI) m/z: 182 (M+1).

### Preparation of compound 1C:

Hydrazine hydrate (832.40 mg) was added to a solution of 1B (2.87 g) in methanol (6 mL) at 0 °C, followed by heating to 90 °C and stirring for 2 h. After the reaction was completed, the reaction mixture was concentrated to dryness under reduced pressure, followed by addition of 5 mL of a mixed solution (ethyl acetate/petroleum ether = 1/10, v/v), and the resultant mixture was stirred for 5 min and then filtered to give solid compound 1C. LCMS (ESI) m/z: 151 (M+1).

### Preparation of compound ID:

DMF (987.06 mg) was slowly dropwise added to phosphorus oxychloride (2.07 g) at 0 °C, followed by stirring for 10 min, with white solid precipitated. Compound 1C (1.2 g) was dissolved in phosphorus oxychloride (30 mL), and the resultant solution was added to the above solid. The reaction mixture was then warmed to 50 °C, added with hydroxylamine hydrochloride (1.67 g) and stirred at 50 °C for 2 h. After the reaction was completed, the reaction mixture was distilled under reduced pressure to remove excess phosphorus oxychloride, and the residue was dissolved in DMF and purified by reversed-phase flash chromatography (Agilent, C18 reversed-phase column, 20-35 µm, 0.1% aqueous formic acid solution/acetonitrile) to give compound ID. LCMS(ESI)m/z: 194(M+1); ¹HNMR(METHANOL-d4) δ ppm 7.87-7.92(m, 1H), 3.02-3.06(m, 2H), 2.70-2.78(m, 2H), 1.99-2.07(m, 2H).

### Preparation of compound IE:

Compound ID (1.6 g), 3,4-dihydro-2*H*-pyran (1.04 g) and trifluoroacetic acid (94.22 mg) were refluxed in tetrahydrofuran (20 mL) for 5 h. After the reaction was completed, the reaction mixture was cooled to room temperature, added with water (20 mL), extracted with ethyl acetate (50 mL × 2), washed with saturated brine (10 mL × 3), dried over anhydrous sodium sulfate, filtered and dried by rotary evaporation, and the residue was purified by column chromatography (1000 mesh silica gel, petroleum ether/ethyl acetate = 100/1 to 5/1) to give compound IE. LCMS(ESI)m/z: 278(M+1); ¹HNMR(CHLOROFORM-d) δ ppm 7.79-7.96(m, 1H), 5.25-5.32(m, 1H), 4.08-4.15(m, 1H), 3.66-3.79(m, 1H), 2.94-3.03(m, 2H), 2.65-2.78(m, 2H), 2.03-2.19(m, 6H), 1.73-1.79(m, 2H).

### Preparation of compound 1G:

Sodium (156 mg) was added to ethanol (9 mL) at room temperature, and after sodium completely disappeared, compound IE (1.0 g) and compound IF (616 mg) were added to the resultant solution and refluxed for 5 h. After the reaction was completed, the reaction mixture was concentrated to dryness under reduced pressure, and the residue was purified by reversed-phase flash chromatography (Agilent, C18 reversed-phase column, 20-35 µm, 0.1% aqueous formic acid solution/acetonitrile) to give compound 1G. LCMS(ESI)m/z: 333(M+1); ¹HNMR(METHANOL-d4) δ ppm 7.99(s, 1H), 5.27-5.46(m, 1H), 4.10-4.13(m, 1H), 3.97-4.09(m, 1H), 3.68-3.81(m, 1H), 2.94-3.15(m, 2H), 2.66-2.82(m, 2H), 1.92-2.17(m, 5H), 1.64(brs, 3H).

### Preparation of compound 1I:

To a solution of compound 1G (313.41 mg) and compound 1H (0.3 g) in dichloromethane (10 mL) was added diisopropylethylamine (349.92 mg) at 20 °C under nitrogen atmosphere. The reaction mixture was stirred at 20 °C for 5 h. After the reaction was completed, the reaction mixture was concentrated to dryness to give crude compound 1I for direct use in the next step. LCMS (ESI) m/z: 470 (M+1).

### Preparation of compound 1J:

Compound 1I (0.4238 g) was dissolved in a mixed solvent of methanol (10 mL) and water (10 mL) at room temperature, followed by addition of sodium hydroxide (360.96 mg). The resultant mixture was warmed to 70 °C and stirred for 10 min. After the reaction was completed, the reaction mixture was distilled to remove methanol, added with water (10 mL), extracted with ethyl acetate (50 mL × 2), washed with saturated brine (10 mL × 2), dried over anhydrous sodium sulfate, filtered and dried by rotary evaporation, and the residue was purified by reversed-phase flash chromatography (Agilent, C18 reversed-phase column, 20-35 µm, 0.1% aqueous formic acid solution/acetonitrile) to give compound 1J. LCMS (ESI) m/z: 452 (M+1).

### Preparation of compound 1-1 hydrochloride and compound 1-2 hydrochloride:

To a solution of compound 1J (0.23 g) in dichloromethane (6 mL) was added dropwise trifluoroacetic acid (4 mL) at room temperature, followed by stirring at room temperature for 1 h. After the reaction was completed, the reaction mixture was concentrated, and the residue was purified by reversed-phase flash chromatography (Agilent, C18 reversed-phase column, 20-35 µm, 0.1% aqueous formic acid/acetonitrile). The resultant product was separated by SFC (column: Daicel OD (250 mm × 30 mm,10 µm); mobile phase: carbon dioxide as phase A, ethanol containing 0.1% aqueous ammonia as phase B; elution gradient: isocratic elution with 50% phase B, duration for each injection: 4 min), and the resultant two fractions were separately re-purified by reversed-phase flash chromatography (Agilent, C18 reversed-phase column, 20-35 µm, 0.1% aqueous hydrochloric acid/acetonitrile) to give compound 1-1 hydrochloride and compound 1-2 hydrochloride.

Determined using the SFC analytical method below, the retention times of the hydrochloride of compound 1-1 hydrochloride and compound 1-2 hydrochloride were 2.200 min and 2.663 min, respectively.

SFC analytical method:
Column: Daicel OD-350 × 4.6 mm I.D., 3 µm; mobile phase: carbon dioxide as phase A, ethanol containing 0.05% diethylamine as phase B; gradient elution: 5-40% phase B; flow rate: 3 mL/min; wavelength: 220 nm; column temperature: 35 °C; back pressure: 100 Bar.

Compound 1-1 hydrochloride: LCMS(ESI)m/z: 368(M+1); ¹HNMR(DMSO-d6) δ ppm 12.37-12.95(m, 1H), 9.96-10.23(m, 1H), 7.98(s, 1H), 4.76-4.83(m, 1H), 3.64-3.73(m, 1H), 3.41-3.42(m, 1H), 3.30-3.39(m, 2H), 3.16-3.25(m, 1H), 3.08(brs, 3H), 2.34-2.43(m, 1H), 2.11-2.27(m, 2H), 1.96-2.09(m, 2H), 1.74-1.95(m, 4H). Compound 1-2 hydrochloride: LCMS(ESI)m/z: 368(M+1); ¹HNMR(DMSO-d6) δ ppm 12.44-13.09(m, 1H), 9.87-10.10(m, 1H), 7.98(s, 1H), 4.73-4.82(m, 1H), 3.69-3.80(m, 1H), 3.45-3.58(m, 1H), 3.28-3.42(m, 2H), 3.13-3.25(m, 1H), 3.04-3.13(m, 3H), 2.36-2.44(m, 1H), 2.12-2.25(m, 2H), 1.94-2.04(m, 2H), 1.74-1.93(m, 4H).

### Example 2. Compound 2-1 Hydrochloride and Compound 2-2 Hydrochloride

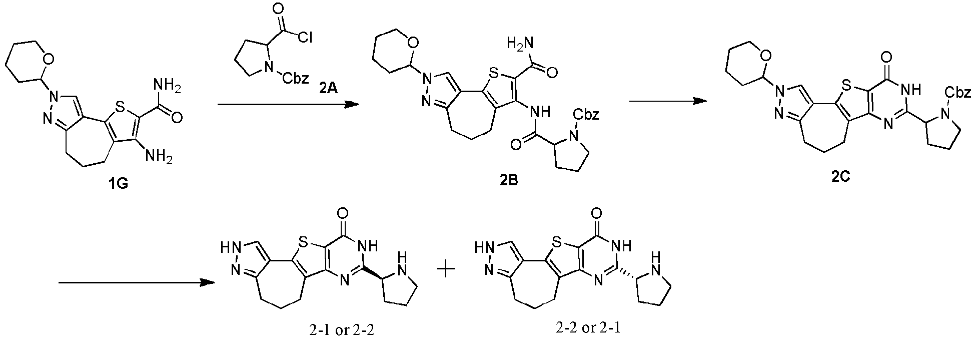

### Preparation of compound 2B:

The compound was prepared as described for compound **1I**. LCMS (ESI) m/z: 564 (M+1).

### Preparation of compound 2C:

The compound was prepared as described for compound **1J.** LCMS (ESI) m/z: 546 (M+1).

### Preparation of compound 2-1 hydrochloride and compound 2-2 hydrochloride:

To compound 3B (0.1 g) was added dropwise a 33% solution of hydrobromic acid in acetic acid (3 mL) at room temperature, followed by stirring at room temperature for 1 h. After the reaction was completed, the reaction mixture was concentrated, and the residue was purified by reversed-phase flash chromatography (Agilent, C18 reversed-phase column, 20-35 µm, 0.1% aqueous formic acid/acetonitrile) and then separated by SFC (column: Daicel AD (250 mm × 30 mm,10 µm); mobile phase: carbon dioxide as phase A, ethanol containing 0.1% aqueous ammonia as phase B; elution gradient: isocratic elution with 50% phase B, duration for each injection: 5.6 min). The resultant two fractions were separately re-purified by reversed-phase flash chromatography (Agilent, C18 reversed-phase column, 20-35 µm, 0.1% aqueous hydrochloric acid/acetonitrile) to give compound 2-1 hydrochloride and compound 2-2 hydrochloride.

Determined using the SFC analytical method below, the retention times of compound 2-1 and compound 2-2 were 0.932 min and 1.421 min, respectively.

SFC analytical method:
Column: Daicel OD-350 × 4.6 mm I.D., 3 µm; mobile phase: carbon dioxide as phase A, ethanol containing 0.05% diethylamine as phase B; gradient elution: isocratic elution with 40% phase B; flow rate: 3 mL/min; wavelength: 220 nm; column temperature: 35 °C; back pressure: 100 Bar.

### Compound 2-1 hydrochloride

LCMS(ESI)m/z: 328(M+1); ¹HNMR(DMSO+D2O) δ ppm 7.93(s, 1H), 4.63-4.72(m, 1H), 3.41-3.53(m, 1H), 3.27-3.38(m, 1H), 3.09-3.20(m, 2H), 2.95-3.07(m, 2H), 2.39-2.50(m, 1H), 1.99-2.12(m, 3H), 1.87-1.99(m, 2H).

### Compound 2-2 hydrochloride

LCMS(ESI)m/z: 328(M+1); HNMR(DMSO+D2O) δ ppm 7.93(s, 1H), 4.63-4.72(m, 1H), 3.41-3.53(m, 1H), 3.27-3.38(m, 1H), 3.09-3.20(m, 2H), 2.95-3.07(m, 2H), 2.39-2.50(m, 1H), 1.99-2.12(m, 3H), 1.87-1.99(m, 2H).

### Example 3. Compound 3-1 Hydrochloride and Compound 3-2 Hydrochloride

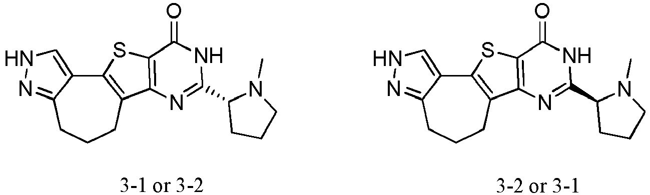

### Preparation of compound 3-1 hydrochloride:

Compound 2-2 hydrochloride (50.00 mg), 37% aqueous formaldehyde (57 µL) and sodium cyanoborohydride (47.99 mg) were stirred in methanol 10 (mL) for 1 h. After the reaction was completed, the reaction mixture was concentrated, and the residue was purified by preparative high performance liquid chromatography (column: Phenomenex Synergi C18 150 × 25 × 10 µm; mobile phase: 0.05% aqueous hydrochloric acid-acetonitrile; acetonitrile gradient: 6-26%, duration: 12 min) to give compound 3-1 hydrochloride. LCMS(ESI)m/z: 342(M+1); ¹HNMR(400MHz, DMSO+D2O) δ ppm 7.94(s, 1H), 4.55(brt, J=7.94Hz, 1H), 3.73-3.77(m, 1H), 3.24-3.37(m, 1H), 3.10-3.23(m, 2H), 3.03-3.08(m, 2H), 2.98(s, 3H), 2.67(brd, J=7.13Hz, 1H), 2.16(brd, J=7.75Hz, 1H), 1.91-2.08(m, 4H).

### Preparation of compound 3-2 hydrochloride:

Compound 3-2 hydrochloride was prepared from compound 2-1 hydrochloride, as described for compound 3-1 hydrochloride. LCMS(ESI)m/z: 342(M+1); ¹HNMR(400MHz, DMSO+D2O) δ ppm 7.96(s, 1H), 4.55(brt, J=7.94Hz, 1H), 3.77-3.82(m, 1H), 3.31(m, 1H), 3.09-3.25(m, 2H), 3.04-3.09(m, 2H), 2.99(s, 3H), 2.63-2.71(m, 1H), 1.93-2.18(m, 5H).

Determined using the SFC analytical method below, the retention times of compound 3-1 hydrochloride and compound 3-2 were 1.879 min and 1.788 min, respectively.

SFC analytical method:
Column: Cellucoat OD-350 × 4.6 mm I.D., 3 µm; mobile phase: carbon dioxide as phase A, methanol containing 0.05% diethylamine as phase B; gradient elution: 5-40% phase B; flow rate: 3 mL/min; wavelength: 220 nm; column temperature: 35 °C; back pressure: 100 Bar.

### Example 4. Compound 4-1 Hydrochloride and Compound 4-2 Hydrochloride

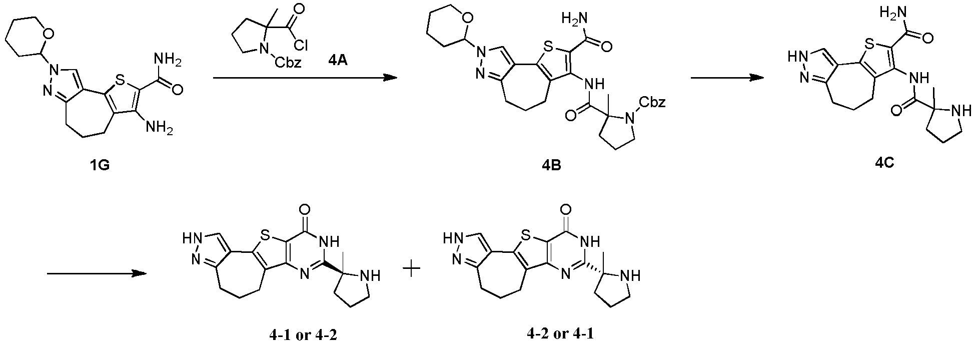

### Preparation of compound 4B:

The compound was prepared as described for compound **1I**. LCMS (ESI) m/z: 578.

### Preparation of compound 4C:

To compound 4B (0.27 g) was added dropwise a 33% solution of hydrobromic acid in acetic acid (2 mL) at room temperature, followed by stirring at room temperature for 1 h. After the reaction was completed, the reaction mixture was concentrated to give compound 3C (bright yellow oil, 170 mg) for direct use in the next step. LCMS (ESI) m/z: 360.

### Preparation of compound 4-1 hydrochloride and compound 4-2 hydrochloride:

Compound 4C (170 mg) and sodium hydroxide (189 mg) were stirred in a mixed solution of methanol (3 mL) and water (1 mL) at 70 °C for 0.5 h. After the reaction was completed, the reaction mixture was concentrated, and the residue was purified by preparative high performance liquid chromatography (column: Phenomenex Synergi C18 150 × 25 × 10 µm; mobile phase: 0.05% aqueous hydrochloric acid-acetonitrile; acetonitrile gradient: 8-28%, duration: 11 min) and then separated by SFC (column: Daicel IG (250 mm × 50 mm,10 µm); mobile phase: carbon dioxide as phase A; methanol solution containing 0.1% aqueous ammonia as phase B; elution gradient: isocratic elution with 55% phase B; duration for each injection: 4.0 min) to give two fractions. Fraction 1 was re-purified by preparative high performance liquid chromatography (column: Phenomenex Synergi C18 150 × 25 × 10 µm; mobile phase: 0.05% aqueous hydrochloric acid-acetonitrile; acetonitrile gradient: 7-27%, duration: 11 min) to give compound 4-1 hydrochloride (100% ee), and fraction 2 was used as compound 4-2 (99.220% ee) without purification.

Compound 4-1 hydrochloride: LCMS(ESI)m/z: 342(M+1); ¹HNMR(400MHz, DMSO-d6) δ ppm 12.77(brs, 1H), 10.07(brs, 1H), 9.09(brs, 1H), 7.97(s, 1H), 3.40(brd, J=5.01Hz, 2H), 3.19(brd, J=2.81Hz, 2H), 3.08(brd, J=5.26Hz, 2H), 2.33-2.39(m, 1H), 2.18-2.25(m, 1H), 2.03-2.11(m, 1H), 1.98(brs, 2H), 1.83-1.91(m, 1H), 1.78(s, 3H).

Compound 4-2: LCMS(ESI)m/z: 342(M+1); ¹HNMR(400MHz, DMSO-d6) δ ppm 7.93(s, 1H), 3.24(brd, J=8.07Hz, 1H), 3.11-3.18(m, 3H), 3.05-3.09(m, 2H), 2.64-2.73(m, 1H), 2.35-2.43(m, 1H), 1.92-2.04(m, 4H), 1.78(brd, J=7.09Hz, 1H), 1.65(s, 3H).

Determined using the SFC analytical method below, the retention times of compound 4-1 hydrochloride and compound 4-2 were 1.777 min and 2.687 min, respectively.

SFC analytical method:
Column: Daicel IG-350 × 4.6 mm I.D., 3 µm; mobile phase: carbon dioxide as phase A, methanol containing 0.05% diethylamine as phase B; gradient elution: isocratic elution with 40% phase B; flow rate: 3 mL/min; wavelength: 220 nm; column temperature: 35 °C; back pressure: 100 Bar.

### Example 5. Compound 5-1 Hydrochloride and Compound 5-2 Hydrochloride

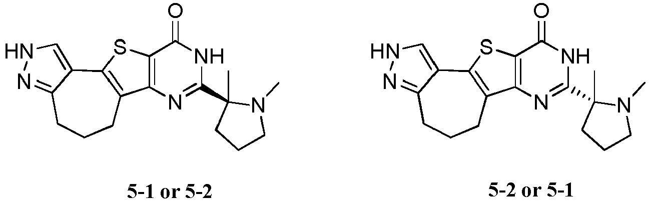

### Preparation of compound 5-1 hydrochloride and compound 5-2 hydrochloride:

The compounds were prepared from compound 4-1 hydrochloride and compound 4-2 hydrochloride, respectively, as described in Example 3.

Compound 5-1 hydrochloride: LCMS(ESI)m/z: 356(M+1); ¹HNMR(400MHz, METHANOL-d4) δ ppm 8.08(s, 1H), 3.89-4.05(m, 1H), 3.45-3.55(m, 1H), 3.34-3.43(m, 1H), 3.11-3.28(m, 6H), 2.51-2.64(m, 1H), 2.38-2.49(m, 1H), 2.32(brd, J=8.80Hz, 1H), 2.05-2.22(m, 3H), 1.85(s, 3H).

Compound 5-2 hydrochloride: LCMS(ESI)m/z: 356(M+1); ¹HNMR(400MHz, METHANOL-d4) δ ppm 8.17(s, 1H), 3.89-4.02(m, 1H), 3.45-3.54(m, 1H), 3.35-3.42(m, 1H), 3.10-3.27(m, 6H), 2.51-2.64(m, 1H), 2.38-2.49(m, 1H), 2.32(brd, J=8.80Hz, 1H), 2.08-2.21(m, 3H), 1.85(s, 3H).

Determined using the SFC analytical method below, the retention times of compound 5-1 hydrochloride and compound 5-2 hydrochloride were 2.148 min and 1.907 min, respectively.

SFC analytical method:
Column: Daicel OD-350 × 4.6 mm I.D., 3 µm; mobile phase: carbon dioxide as phase A, methanol containing 0.05% diethylamine as phase B; gradient elution: 5-40% phase B; flow rate: 3 mL/min; wavelength: 220 nm; column temperature: 35 °C; back pressure: 100 Bar.

### Example 6. Compound 6-1 Hydrochloride and Compound 6-2 Hydrochloride

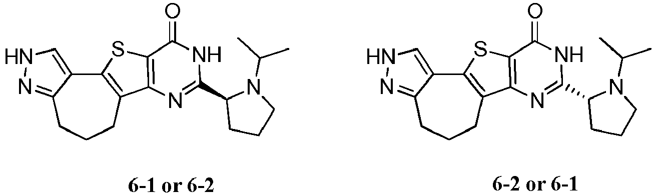

### Preparation of compound 6-1 hydrochloride:

Example 2-1(95 mg), acetone (107 µL) and sodium cyanoborohydride (91 mg) were stirred in methanol (10 mL) at room temperature for 1 h. After the reaction was completed, the reaction mixture was concentrated, and the residue was purified by preparative high performance liquid chromatography (column: Phenomenex synergy C18 150 × 25 × 10 µm; mobile phase: 0.05% aqueous hydrochloric acid-acetonitrile; acetonitrile gradient: 11-31%, duration: 11 min) to give compound 6-1 hydrochloride. LCMS(ESI)m/z: 370(M+1); ¹HNMR(400MHz, DMSO-d6) δ ppm 12.75-13.15(m, 1H), 9.63(brs, 1H), 7.97(s, 1H), 4.72(brd, J=7.70Hz, 1H), 3.82(brdd, J=6.48, 4.03Hz, 1H), 3.67-3.73(m, 1H), 3.33-3.47(m, 1H), 3.19-3.33(m, 1H), 3.04-3.15(m, 3H), 2.56-2.70(m, 1H), 1.92-2.18(m, 5H), 1.23-1.35(m, 6H).

### Preparation of compound 6-2 hydrochloride:

Compound 6-2 hydrochloride was prepared from compound 2-2 hydrochloride, as described for compound 6-1 hydrochloride. LCMS(ESI)m/z: 370(M+1); ¹HNMR(400MHz, DMSO-d6) δ ppm 12.59-13.14(m, 1H), 9.63(brs, 1H), 7.97(s, 1H), 4.63-4.77(m, 1H), 3.82(brdd, J=6.48, 3.79Hz, 1H), 3.71(brd, J=6.36Hz, 1H), 3.34-3.49(m, 1H), 3.19-3.33(m, 1H), 3.03-3.14(m, 3H), 2.53-2.66(m, 1H), 2.18-2.36(m, 1H), 1.90-2.15(m, 5H), 1.32(d, J=6.60Hz, 3H), 1.26(d, J=6.60Hz, 3H).

Determined using the SFC analytical method below, the retention times of compound 6-1 hydrochloride and compound 6-2 hydrochloride were 1.838 min and 1.992 min, respectively.

SFC analytical method:
Column: Daicel OD-350 × 4.6 mm I.D., 3 µm; mobile phase: carbon dioxide as phase A, methanol containing 0.05% diethylamine as phase B; gradient elution: 5-40% phase B; flow rate: 3 mL/min; wavelength: 220 nm; column temperature: 35 °C; back pressure: 100 Bar.

### Example 7. Compound 7-1 Formate and Compound 7-2 Formate

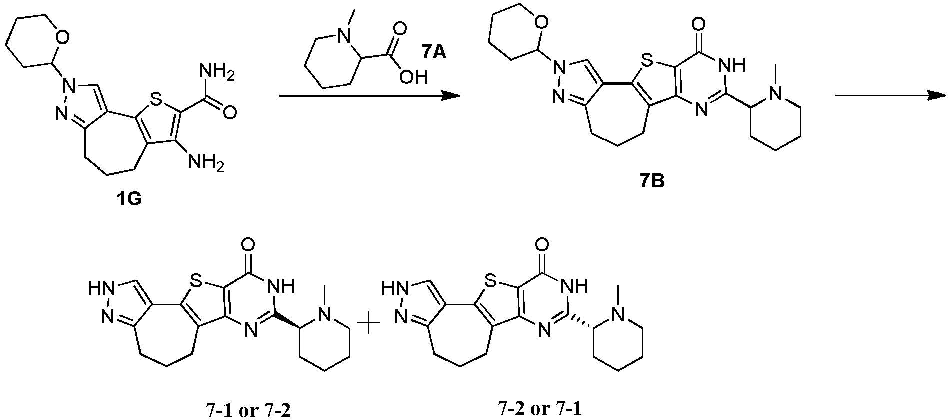

### Preparation of compound 7B:

To a solution of compound **7A** (91.72 mg) and diisopropylethylamine (106.99 mg) in dichloromethane (2 mL) was added 2,2-dicarbonylimidazole (44.74 mg) at room temperature, followed by stirring at room temperature for 2 h. The reaction mixture was distilled to remove the solvent, and the residue was added with compound 6A (100 mg) and *N*,*N*-dimethylformamide (2 mL), followed by stirring at 100 °C for 48 h. After the reaction was completed, the reaction mixture was concentrated to dryness, and the residue was purified by reversed-phase flash chromatography (Agilent, C18 reversed-phase column, 20-35 µm, 0.1% aqueous formic acid solution/acetonitrile) to give compound 7B formate. LCMS (ESI) m/z: 440.

### Preparation of compound 7-1 formate and compound 7-2 formate:

The compounds were prepared as described in Example 1.

Compound 7-1 formate: LCMS(ESI)m/z: 356(M+1); ¹HNMR(METHANOL-d4) δ ppm 8.09(s, 1H), 4.20-4.23(m, 1H), 3.67-3.76(m, 1H), 3.29(brd, J=4.0Hz, 2H), 3.18(brd, J=5.7Hz, 2H), 2.97(s, 3H), 2.32-2.41(m, 1H), 2.10-2.20(m, 2H), 1.99(s, 5H), 1.69-1.85(m, 1H).

Compound 7-2 formate: LCMS(ESI)m/z: 356(M+1); ¹HNMR(METHANOL-d4) δ ppm 8.06(s, 1H), 4.19-4.27(m, 1H), 3.67-3.76(m, 1H), 3.29(brd, J=4.0Hz, 2H), 3.18(brd, J=5.7Hz, 2H), 2.97(s, 3H), 2.32-2.41(m, 1H), 2.10-2.20(m, 2H), 1.99(s, 5H), 1.69-1.85(m, 1H).

Determined using the SFC analytical method below, the retention times of compound 7-1 formate and compound 7-2 formate were 0.943 min and 1.326 min, respectively.

SFC analytical method:
Column: Chiralpak AD-350 × 4.6 mm I.D., 3 µm; mobile phase: carbon dioxide as phase A, ethanol containing 0.05% diethylamine as phase B; gradient elution: isocratic elution with 40% phase B; flow rate: 3 mL/min; wavelength: 220 nm; column temperature: 35 °C; back pressure: 100 Bar.

### Example 8. Compound 8-1 Hydrochloride and Compound 8-2 Hydrochloride

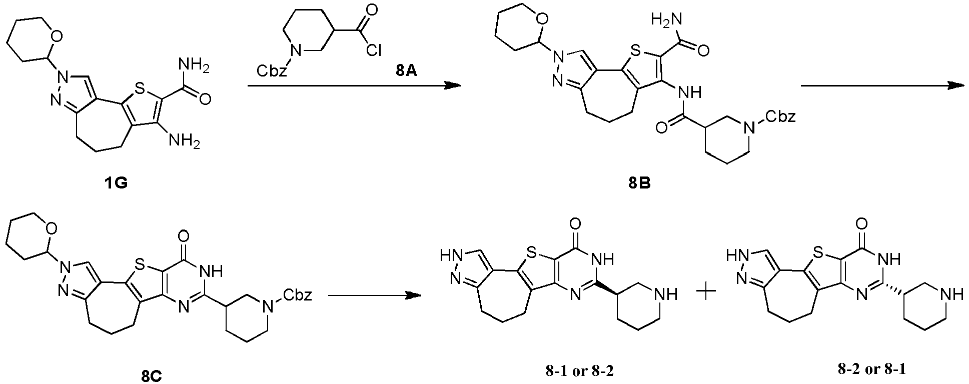

### Preparation of compound 8B:

The compound was prepared as described for compound **1I**. LCMS (ESI) m/z: 578.

### Preparation of compound 8C:

The compound was prepared as described for compound **1J.** LCMS (ESI) m/z: 560.

### Preparation of compound 8-1 hydrochloride and compound 8-2 hydrochloride:

Prepared as described in Example **2.**

Compound 8-1 hydrochloride: LCMS(ESI)m/z: 342(M+1); ¹HNMR(400MHz, DMSO-d6) δ ppm 12.47(brs, 1H), 9.12-9.39(m, 2H), 7.94(s, 1H), 3.47(brd, J=11.00Hz, 1H), 3.15-3.30(m, 3H), 3.02-3.12(m, 4H), 2.87-2.96(m, 1H), 2.14(brd, J=10.15Hz, 1H), 1.93-2.02(m, 2H), 1.75-1.88(m, 2H), 1.61-1.72(m, 1H). Compound 8-2 hydrochloride: LCMS(ESI)m/z: 342(M+1); ¹HNMR(400MHz, DMSO-d6) δ ppm 12.45(brs, 1H), 9.05-9.47(m, 2H), 7.95(s, 1H), 3.47(brd, J=10.88Hz, 1H), 3.15-3.31(m, 3H), 3.01-3.13(m, 4H), 2.85-2.97(m, 1H), 2.14(brd, J=9.78Hz, 1H), 1.92-2.02(m, 2H), 1.75-1.89(m, 2H), 1.63-1.72(m, 1H). Determined using the SFC analytical method below, the retention times of compound 8-1 hydrochloride and compound 8-2 hydrochloride were 0.902 min and 1.802 min, respectively.

SFC analytical method:
Column: Chiralpak AD-350 × 4.6 mm I.D., 3 µm; mobile phase: carbon dioxide as phase A, ethanol containing 0.05% diethylamine as phase B; gradient elution: isocratic elution with 40% phase B; flow rate: 3 mL/min; wavelength: 220 nm; column temperature: 35 °C; back pressure: 100 Bar.

### Example 9. Compound 9-1 Hydrochloride and Compound 9-2 Hydrochloride

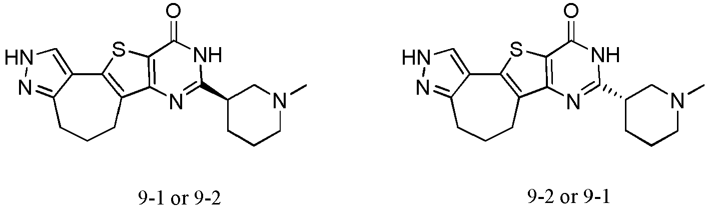

### Preparation of compound 9-1 hydrochloride and compound 9-2 hydrochloride:

The compounds were prepared from compound 8-1 hydrochloride and compound 8-2 hydrochloride, respectively, as described in Example **3-1.**

Compound 9-1 hydrochloride: LCMS(ESI)m/z: 356(M+1); ¹HNMR(400MHz, DMSO-d6) δ ppm 10.97(brs, 1H), 7.95(s, 1H), 3.57-3.64(m, 1H), 3.29-3.43(m, 3H), 3.04-3.12(m, 4H), 2.90-2.99(m, 1H), 2.80(d, J=4.65Hz, 3H), 2.17(brd, J=13.45Hz, 1H), 1.87-1.99(m, 3H), 1.36-1.69(m, 1H).

Compound 9-2 hydrochloride: LCMS(ESI)m/z: 356(M+1); ¹HNMR(400MHz, DMSO-d6) δ ppm 10.97(brs, 1H), 7.95(s, 1H), 3.61(brd, J=7.34Hz, 1H), 3.31-3.42(m, 3H), 3.03-3.10(m, 4H), 2.90-2.99(m, 1H), 2.80(d, J=4.77Hz, 3H), 2.17(brd, J=13.45Hz, 1H), 1.90-2.00(m, 4H), 1.43-1.64(m, 1H).

Determined using the SFC analytical method below, the retention times of compound 9-1 hydrochloride and compound 9-2 hydrochloride were 2.183 min and 1.681 min, respectively.

SFC analytical method:
Column: Chiralpak IC-350 × 4.6 mm I.D., 3 µm; mobile phase: carbon dioxide as phase A, ethanol containing 0.05% diethylamine as phase B; gradient elution: isocratic elution with 40% phase B; flow rate: 3 mL/min; wavelength: 220 nm; column temperature: 35 °C; back pressure: 100 Bar.

### Example 10. Compound 10 Hydrochloride

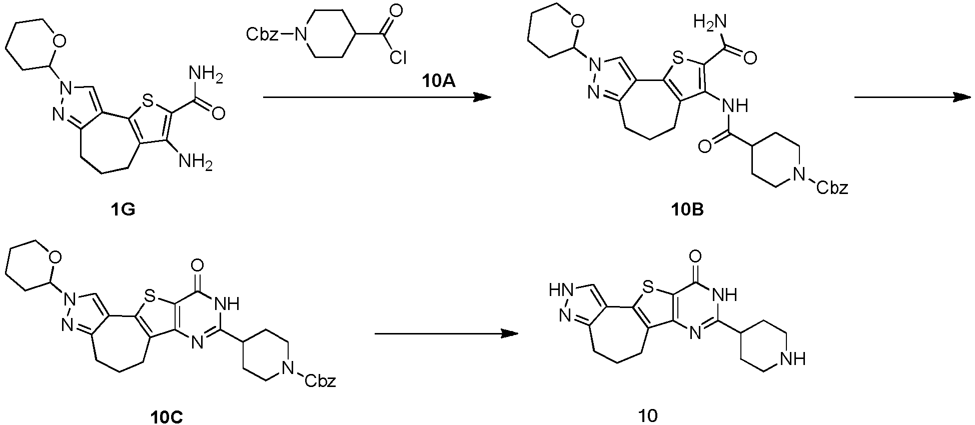

### Preparation of compound 10B:

The compound was prepared as described for compound **1I**. LCMS (ESI) m/z: 578.

### Preparation of compound 10C:

The compound was prepared as described for compound 1J. LCMS (ESI) m/z: 560.

### Preparation of compound 10 hydrochloride:

The compound was prepared as described for compound 2-1 hydrochloride. LCMS(ESI)m/z: 342(M+1); ¹HNMR(400MHz, DMSO-d6) δ ppm 12.12 - 12.63 (m, 1 H), 9.01 (br d, J=8.93 Hz, 1 H), 8.65 (br d, J=9.78 Hz, 1 H), 7.94 (s, 1 H), 3.40 (br d, J=6.72 Hz, 1 H), 2.91 - 3.08 (m, 7 H), 1.91 - 2.17 (m, 7 H).

### Example 11. Compound 11 Hydrochloride

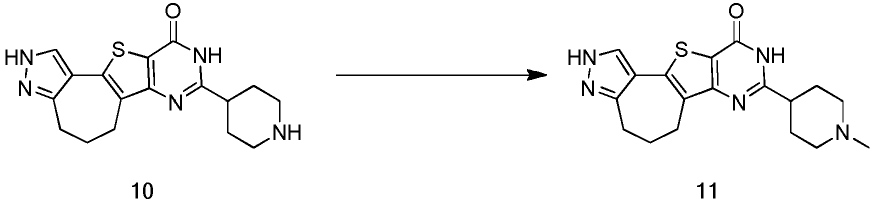

### Preparation of compound 11 hydrochloride:

The compound was prepared from compound 10 hydrochloride, as described in Example **3-1.** LCMS(ESI)m/z: 356(M+1); ¹HNMR(400MHz, DMSO-d6) δ ppm 12.37(brs, 1H), 10.41(brs, 1H), 7.84, -8.07(m, 1H), 3.47-3.56(m, 1H), 3.49(brd, J=11.25Hz, 1H), 3.19-3.36(m, 1H), 2.96-3.17(m, 6H), 2.82-2.95(m, 1H), 2.76(d, J=4.65Hz, 3H), 2.05-2.32(m, 4H), 1.91-2.04(m, 2H), 1.06(t, J=7.03Hz, 1H).

### Example 12. Compound 12 Hydrochloride

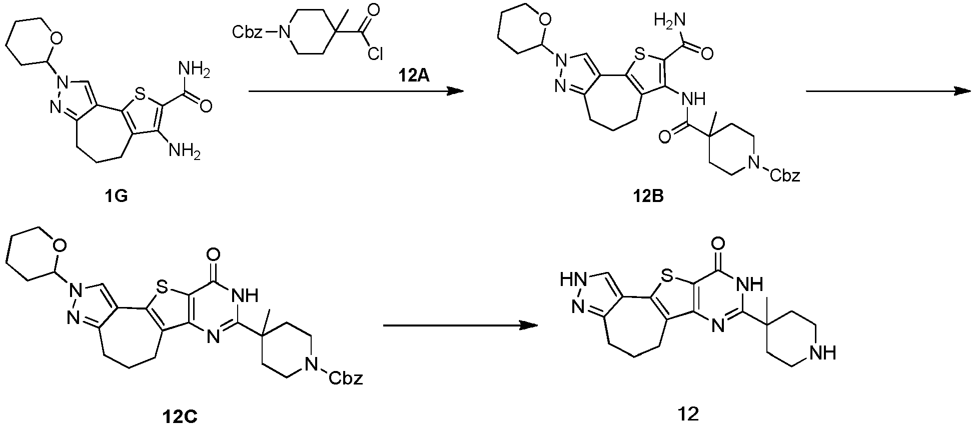

### Preparation of compound 12 hydrochloride:

The compound was prepared as described for compound 2-1 hydrochloride. LCMS (ESI) m/z: 356 (M+1). ¹HNMR (400 MHz, DMSO-d₆) δ ppm 12.14 (brs, 1H), 8.78 (brs, 2H), 7.86-8.03 (m, 1H), 2.99-3.26 (m, 8H), 2.52-2.56 (m, 2H), 1.73-2.06 (m, 4H), 1.27-1.42 (m, 3H).

### Example 13. Compound 13 Hydrochloride

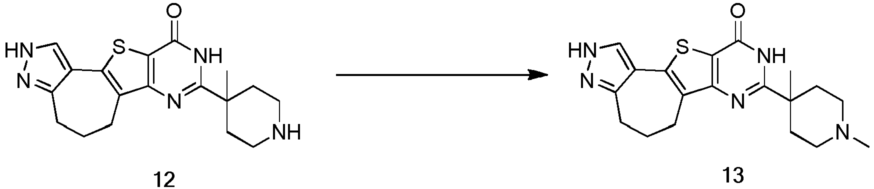

### Preparation of compound 13 hydrochloride:

The compound was prepared from compound 12 **hydrochloride,** as described for compound 3-1 hydrochloride. LCMS(ESI)m/z: 370(M+1); ¹HNMR(400MHz, DMSO-d6) δ ppm 11.94-12.36(m, 1H), 10.16-10.60(m, 1H), 7.82-8.04(m, 1H), 3.32(brd, J=11.98Hz, 2H), 2.98-3.11(m, 5H), 2.61-2.80(m, 4H), 2.09-2.32(m, 2H), 1.81-2.05(m, 4H), 1.44(s, 3H).

### Example 14. Compound 14-1 Hydrochloride and Compound 14-2 Hydrochloride

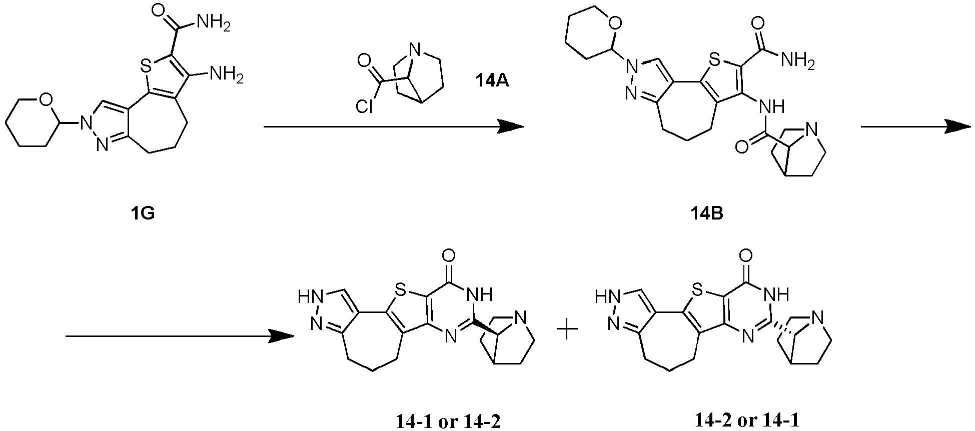

Example 14 was prepared as described in Example 1.

Compound 14-1 hydrochloride: LCMS(ESI)m/z: 354(M+1); ¹HNMR(400MHz, DMSO-d6) δ ppm12.78(brs, 1H), 11.01(brs, 1H), 7.98(s, 1H), 4.88(s, 1H), 3.92(brd, J=2.6Hz, 1H), 3.50(brd, J=2.4Hz, 1H), 3.42-3.29(m, 3H), 3.20-3.03(m, 4H), 2.15(brd, J=2.8Hz, 1H), 2.05-1.87(m, 3H), 1.83-1.71(m, 2H).

Compound 14-2 hydrochloride: LCMS(ESI)m/z: 354(M+1); ¹HNMR(400MHz, DMSO-d6) δ ppm 12.78(brs, 1H), 11.01(brs, 1H), 7.98(s, 1H), 4.88(s, 1H), 3.92(brd, J=2.6Hz, 1H), 3.50(brd, J=2.4Hz, 1H), 3.42-3.29(m, 3H), 3.20-3.03(m, 4H), 2.15(brd, J=2.8Hz, 1H), 2.05-1.87(m, 3H), 1.83-1.71(m, 2H).

Determined using the SFC analytical method below, the retention times of compound 14-1 hydrochloride and compound 14-2 hydrochloride were 1.453 min and 2.828 min, respectively.

SFC analytical method:
Column: Chiralpak AD-350 × 4.6 mm I.D., 3 µm; mobile phase: carbon dioxide as phase A, methanol containing 0.05% diethylamine as phase B; gradient elution: isocratic elution with 40% phase B; flow rate: 3 mL/min; wavelength: 220 nm; column temperature: 35 °C; back pressure: 100 Bar.

### Experimental Example 1. Detection of Inhibitory Effect of Compounds Against Activity of Cdc7/DBF4 Materials:

Cdc7/DBF4 kinase detection kit purchased from Promega; and
Nivo multi-marker analyzer (PerkinElmer).

### Method:

An enzyme, a substrate, adenosine triphosphate and an inhibitor were diluted with the kinase buffer in the kit. A test compound was serially 5-fold diluted to an 8th concentration, i.e., from 10 µM to 0.13 nM, with the DMSO concentration being 5%, and the duplicate well experiment was set up. To a microplate were added 1 µL of inhibitors of various concentration gradients, 2 µL of CDC7/DBF4 kinase (6.25 ng), 2 µL of a mixture of substrate and ATP (10 µM adenosine triphosphate, 0.2 µg/µL substrate), and the final concentration gradient of the compound was diluted from 2 µM to 0.025 nM. The reaction system was left reacting at 25 °C for 60 min. After the reaction was completed, 5 µL of ADP-Glo reagent was added to each well, and the reaction was continued at 25 °C for 40 min. After the reaction was completed, 10 µL of the kinase detection reagent was added to each well. After 30 min of reaction at 25 °C, the chemiluminescence was read using the multi-marker analyzer with an integration time of 0.5 s.

### Data analysis:

The original data were converted to inhibition using the equation **(Sample - Min)/(Max - Min) × 100%,** and the IC₅₀ value was then curve fitted using four parameters (obtained from the "log(inhibitor) vs. response-Variableslope" model in GraphPadPrism). Table 1 provides the enzymatic inhibitory activity of the compounds disclosed herein against Cdc7/DBF4.

**Results:** See Table 1.

**Table 1**

| **Test compounds** | **Cdc7/DBF4 IC₅₀ (nmol)** | **Test compounds** | **Cdc7/DBF4 IC₅₀ (nmol)** |
|---|---|---|---|
| Compound 1-1 hydrochloride | 3.02 | Compound 7-1 formate | 3.07 |
| Compound 1-2 hydrochloride | 1.29 | Compound 7-2 formate | 2.7 |
| Compound 2-1 hydrochloride | 3.73 | Compound 8-1 hydrochloride | 4.27 |
| Compound 2-2 hydrochloride | 1.52 | Compound 8-2 hydrochloride | 1.17 |
| Compound 3-1 hydrochloride | 1.19 | Compound 9-1 hydrochloride | 4.85 |
| Compound 3-2 hydrochloride | 0.42 | Compound 9-2 hydrochloride | 1.09 |
| Compound 4-1 hydrochloride | 4.56 | Compound 10 hydrochloride | 1.69 |
| Compound 4-2 hydrochloride | 6.73 | Compound 11 hydrochloride | 4.85 |
| Compound 5-1 hydrochloride | 2.04 | Compound 12 hydrochloride | 1.38 |
| Compound 5-2 hydrochloride | 2.35 | Compound 13 hydrochloride | 5.66 |
| Compound 6-1 hydrochloride | 1.7 | Compound 14-1 hydrochloride | 1.21 |
| Compound 6-2 hydrochloride | 0.99 | Compound 14-2 hydrochloride | 1.43 |

### Experimental Example 2. Detection of Inhibitory Effect of Compounds Against Activity of Colo205 Cells

### Materials:

1640 medium; fetal bovine serum; penicillin/streptomycin antibiotics purchased from Wisent; CellTiter-Glo (chemiluminescence detection reagent for cell viability) reagent purchased from Promega; COLO205 cell line purchased from Wuhan Procell Life Science&Technology Co., Ltd; and Nivo multi-marker analyzer (PerkinElmer).

### Method:

COLO205 cells were plated on to white 96-well plates by adding 80 µL of cell suspension (containing 3000 COLO205 cells) to each well. The cell plate was incubated in a CO₂ incubator overnight.

A test compound was serially 3-fold diluted to an 8th concentration, i.e., from 2 mM to 920 nM, and the duplicate well experiment was set up. 78 µL of medium was added to an intermediate plate, 2 µL of the serially diluted compound was transferred to corresponding wells of the intermediate plate, and after mixing, the mixture was transferred to the cell plate at 20 µL per well. The concentration of the compound transferred to the cell plate ranged from 10 µM to 4.57 nM. The cell plate was incubated in a CO₂ incubator for 3 days. Another cell plate was provided for reading signal values on the day of compound addition and these values were used as Max values (the Max in the equation below) in data analysis. The chemiluminescence detection reagent for cell viability was added to this cell plate at 25 µL per well and the luminescence signals were stabilized by incubation at room temperature for 10 min. Readings were taken using the multi-marker analyzer. The chemiluminescence detection reagent for cell viability was added to the cell plate at 25 µL per well and the luminescence signals were stabilized by incubation at room temperature for 10 min. Readings were taken using the multi-marker analyzer.

### Data analysis:

The original data were converted to inhibition using the equation **(Sample - Min)/(Max - Min) × 100%,** and the IC₅₀ value was then curve fitted using four parameters (obtained from the "log(inhibitor) vs. response-Variableslope" model in GraphPadPrism). Table 2 provides the inhibitory activity of the compounds disclosed herein against COLO205 cell proliferation.

**Results:** See Table 2.

**Table 2**

| **Test compounds** | **Colo205 IC₅₀ (nmol)** |
|---|---|
| Compound 1-1 hydrochloride | 13.8 |
| Compound 1-2 hydrochloride | 53.62 |
| Compound 14-1 hydrochloride | 50.09 |

### Experimental Example 3. Single-Dose Pharmacokinetic Study in Mice

### Objective:

To evaluate the pharmacokinetic behavior by using male CD-1 mice as test animals and determining the drug concentrations of the compounds in the plasma after single-dose administration.

### Method:

Healthy adult male CD-1 mice were selected for intragastric administration. A compound was mixed with an appropriate amount of 5% DMSO/95% (10% hydroxypropyl-β-cyclodextrin), vortexed and sonicated to prepare a 1 mg/mL clear solution for later use. After the mice were administered intravenously at 2 mg/kg and orally at 10 mg/kg, whole blood was collected at certain time points, and plasma was separated. After pretreatment of the samples, the drug concentration was measured by LC-MS/MS, and pharmacokinetic parameters were calculated using Phoenix WinNonlin software.

**Results:** See Table 3.

**Table 3. Pharmacokinetic results in mice**

| **Pharmacokinetic results (IV: 2 mg/kg; PO: 10 mg/kg)** | **Compound 1-1 hydrochloride** |
|---|---|
| Clearance (mL/min/kg) | 35.2 |
| Apparent volume of distribution (L/kg) | 4.13 |
| AUC₀₋ₗₐₛₜ (intravenous injection, nM. h) | 2454 |
| AUC₀₋ₗₐₛₜ (oral, nM. h) | 9080 |
| Half-life (h) | 4.03 |
| Maximum concentration (nM) | 2230 |
| Bioavailability (%) | 71.4 |

### Experimental Example 4. In Vivo Pharmacodynamic Study of Compounds in Subcutaneous Xenograft Tumor Nude Mouse Model of Human Colorectal Cancer Cell SW620

### Cell culturing:

Human colorectal cancer cell SW620 cells of the 7th passage were cultured in an L-15 medium containing 10% fetal bovine serum, 100 U/mL penicillin and 100 µg/mL streptomycin through *in vitro* monolayer culture in an incubator at 37 °C/0% CO₂ for 4 passages with conventional medium replacement. At a cell saturation of 80-90%, the cells were digested with pancreatin-EDTA, counted and resuspended in PBS at a density of 5 × 10⁶ cells/100 µL.

### Tumor cell inoculation and grouping:

Cell inoculation: Each mouse was inoculated on the right cervical dorsum with 0.1 mL of the cell suspension of 5 × 10⁶ SW620 cells in PBS. At an average tumor volume up to about 134 mm³, the mice were randomly grouped and administrated.

**Table 4. Animal grouping and administration regimen**

| **Groups** | **n** | **Compounds** | **Dosages (mg/kg) and frequencies** | **Volumes (µL/g)** | **Routes** |
|---|---|---|---|---|---|
| 1 | 6 | Vehicle | BID | 10 | p.o. |
| 2 | 6 | TAK-931 | 40 × BID | 10 | p.o. |
| 3 | 6 | Example 1-1 | 2.5 × BID | 10 | p.o. |
| 4 | 6 | Example 1-1 | 5 × BID | 10 | p.o. |
| 5 | 6 | Example 1-1 | 7.5 × BID | 10 | p.o. |

| | | | | | |
|---|---|---|---|---|---|
| Note: Vehicle refers to a vehicle control group. | | | | | |

### Tumor measurement and experimental indices:

Tumor diameters were measured twice weekly using a vernier caliper. The tumor volume was calculated using the following formula: V = 0.5 a × b², where a and b represent the long diameter and short diameter of the tumor, respectively.

The anti-tumor therapeutic effect of the compound was evaluated by TGI (%) or relative tumor proliferation rate T/C (%). Relative tumor proliferation rate T/C (%) = TRTV / CRTV × 100% (TRTV: RTV of treatment group; CRTV: RTV of negative control group). Relative tumor volumes (RTVs) were calculated from the tumor measurement results using the following formula: RTV = Vt / V0, where V0 is the average tumor volume measured at the time of grouping (i.e., D0), Vt is the average tumor volume measured at a certain measurement, and TRTV and CRTV are taken from the data measured on the same day.

TGI (%), reflects the tumor growth inhibition rate. TGI (%) = [(1 - (average tumor volume at the end of administration in a treatment group - average tumor volume at the start of administration of the treatment group)) / (average tumor volume at the end of treatment of the vehicle control group - average tumor volume at the start of treatment of the vehicle control group)] × 100%.

After day 22 of the grouping, mice were euthanized, plasma and tumors were sampled and tumors were weighed and photographed.

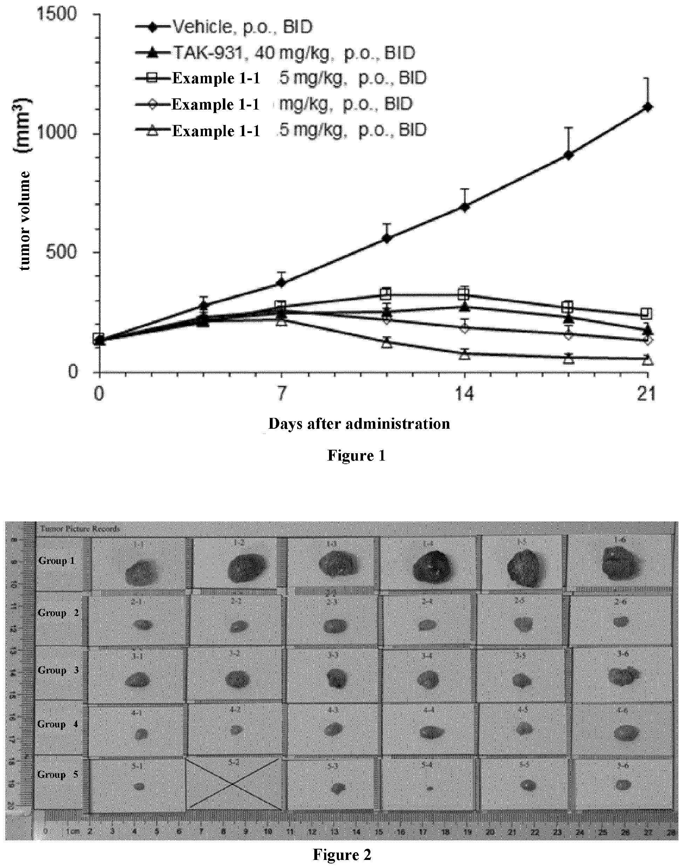

**Results:** Table 5, FIG. 1 and FIG. 2.

**Table 5. Antitumor effect of the compounds on the xenograft tumor model of the human colorectal cancer cell SW620**

| **Groups** | **Tumor volume (mm³)** | **Tumor volume (mm³)** | **RTV** | **TGI (%)** | **T/C (%)** |
|---|---|---|---|---|---|
| | **(Day 1)** | **(Day 21)** | **(Day 21)** | **(Day 21)** | **(Day 21)** |
| Vehicle, p.o, BID | 134±14^{a} | 1112±122 | - | - | - |
| TAK-931, 40 mg/kg, BID | 134±13 | 176±27 | 1.36 | 96 | 16 |
| Compound 1-1 hydrochloride, 2.5 mg/kg BID | 134±13 | 237±27 | 1.88 | 89 | 22 |
| Compound 1-1 hydrochloride, 5 mg/kg BID | 134±15 | 133±32 | 0.93 | 100 | 11 |
| Compound 1-1 hydrochloride, 7.5 mg/kg BID | 134±14 | 53±16 | 0.31 | 108 | 4 |

## Claims

1. A compound of formula (I), an isomer thereof or a pharmaceutically acceptable salt thereof, 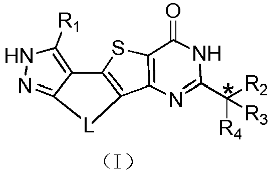 wherein,
the carbon atom with "^{∗}" can be a chiral carbon atom present in a form of a single (*R*) or (*S*) enantiomer or in a form rich in one enantiomer;
L is selected from the group consisting of -CH₂-CH₂-CH₂-, -CH₂-O-CH₂-, -CH₂-S-CH₂-, -CH₂-NH-CH₂-, -NH-CH₂-CH₂-, -S-CH₂-CH₂- and -O-CH₂-CH₂-;
R₁ is selected from the group consisting of H, halogen, CN, C₁₋₆ alkyl, C₃₋₆ cycloalkyl, phenyl, and 5-6 membered heteroaryl, wherein the C₁₋₆ alkyl, C₃₋₆ cycloalkyl, phenyl, and 5-6 membered heteroaryl are each independently optionally substituted with 1, 2 or 3 Rₐ, the 5-6 membered heteroaryl containing 1, 2 or 3 heteroatoms or heteroatom groups each independently selected from the group consisting of O, S, N and NH; R₂ is selected from R_{b}, R₃ is selected from NH₂, and R₄ is selected from H;
alternatively, R₂ is selected from R_{c}, and R₃ and R₄ are joined to form a ring A group optionally substituted with 1, 2 or 3 Rₑ, wherein the ring A group is selected from the group consisting of C₆₋₁₄ aryl, 5-14 membered heteroaryl, 5-12 membered heterocycloalkenyl and 4-14 membered heterocycloalkyl each independently containing 1, 2 or 3 heteroatoms or heteroatom groups independently selected from the group consisting of O, S, N and NR_{d};
Rₐ is each independently selected from the group consisting of F, Cl, Br, I, OH, CN, NH₂, -CH₃ and 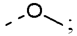
R_{b} is selected from the group consisting of H and C₁₋₆ alkyl, the C₁₋₆ alkyl being optionally substituted with 1, 2 or 3 R_{bb};
R_{c} is selected from the group consisting of H, F, Cl, Br, I and C₁₋₃ alkyl;
R_{d} is selected from the group consisting of H and C₁₋₄ alkyl;
R_{bb} is selected from the group consisting of -OCH₃, -OCH₂CH₃, -O-CH(CH₃)₂, cyclopropyl, cyclopentyl, phenyl, pyrazolyl, pyridyl, NH₂, -NHCH₃ and -N(CH₃)₂;
Rₑ is selected from the group consisting of F, Cl, Br, I, OH, CN, COOH, NH₂, -NHCH₃, -N(CH₃)₂, -CH₃, -CH₂CH₃, -CF₃, -OCH₃, -OCH₂CH₃, -O-CH(CH₃)₂, -C(=O)OCH₃, -C(=O)CH₃ and -C(=O)CH₂CH₃.

2. The compound, the isomer thereof or the pharmaceutically acceptable salt thereof according to claim 1, wherein R₁ is selected from the group consisting of H, F, Cl, Br, I, CN, C₁₋₃ alkyl, C₃₋₅ cycloalkyl, phenyl, and 6 membered heteroaryl, wherein the C₁₋₃ alkyl, C₃₋₅ cycloalkyl, phenyl, and 6 membered heteroaryl are each independently optionally substituted with 1, 2 or 3 Rₐ, and the 6 membered heteroaryl contains 1, 2 or 3 heteroatoms selected from N; preferably, R₁ is selected from the group consisting of H, F, Cl, Br, I, CN, -CH₃, -CH₂CH₃, cyclopropyl, phenyl and pyridyl, wherein the -CH₃, -CH₂CH₃, cyclopropyl, phenyl and pyridyl are each independently optionally substituted with 1, 2 or 3 Rₐ; further preferably, R₁ is selected from the group consisting of H, F, Cl, Br, I, CN, CH₃, CH₂CH₃, CF₃, cyclopropyl, phenyl and pyridyl; further more preferably, R₁ is selected from H.

3. The compound, the isomer thereof or the pharmaceutically acceptable salt thereof according to claim 1, wherein R_{b} is selected from the group consisting of H and C₁₋₄ alkyl, the C₁₋₄ alkyl being optionally substituted with 1, 2 or 3 R_{bb}; preferably, R_{b} is selected from the group consisting of H, methyl, ethyl, isopropyl, *n*-propyl, *n*-butyl and isobutyl; further preferably, R_{b} is selected from C₁₋₃ alkyl; further more preferably, R_{b} is selected from isopropyl.

4. The compound, the isomer thereof or the pharmaceutically acceptable salt thereof according to claim 1, wherein R_{c} is selected from the group consisting of H, F and C₁₋₃ alkyl; preferably, R_{c} is selected from the group consisting of H, methyl, ethyl and F; further preferably, R_{c} is selected from the group consisting of H and methyl.

5. The compound, the isomer thereof or the pharmaceutically acceptable salt thereof according to claim 1, wherein R_{d} is selected from the group consisting of H, methyl, ethyl, *n*-propyl, isopropyl, and *n*-butyl; preferably, R_{d} is selected from the group consisting of H and C₁₋₃ alkyl; further preferably, R_{d} is selected from the group consisting of H, methyl and isopropyl.

6. The compound, the isomer thereof or the pharmaceutically acceptable salt thereof according to claim 1, wherein the ring A group is selected from the group consisting of C₆₋₁₀ aryl, 5-9 membered heteroaryl, 5-7 membered heterocycloalkenyl and 4-10 membered heterocycloalkyl optionally substituted with 1, 2 or 3 Rₑ and each independently containing 1, 2 or 3 heteroatoms or heteroatom groups independently selected from the group consisting of O, S, N and NR_{d}; preferably, the ring A group is selected from 5-9 membered heterocycloalkyl optionally substituted with 1, 2 or 3 Rₑ and containing 1, 2 or 3 heteroatoms or heteroatom groups independently selected from the group consisting of O, S, N and NR_{d}; further preferably, the ring A group is selected from 5-9 membered heterocycloalkyl containing 1 heteroatom or heteroatom group selected from the group consisting of N and NR_{d}.

7. The compound, the isomer thereof or the pharmaceutically acceptable salt thereof according to claim 6, wherein the ring A group is selected from the group consisting of pyrrolidinyl, piperidinyl, morpholinyl, 1-azabicyclo[2.2.2]octanyl, 1-azabicyclo[2.2.1]heptanyl, 1-azabicyclo[3.2.2]nonanyl and azepanyl optionally substituted with 1, 2 or 3 Rₑ, and contains 1 heteroatom or heteroatom group selected from the group consisting of N and NR_{d}; preferably, the ring A group is selected from the group consisting of

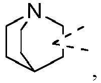

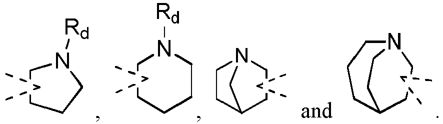

8. The compound, the isomer thereof or the pharmaceutically acceptable salt thereof according to claim 1, wherein the structural unit 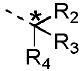 is selected from 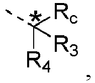 further from the group consisting of

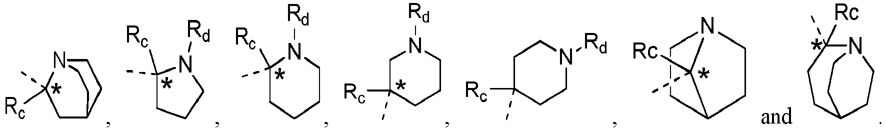

9. The compound, the isomer thereof or the pharmaceutically acceptable salt thereof according to claim 8, wherein the structural unit 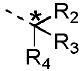 is selected from 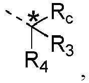 further from the group consisting of 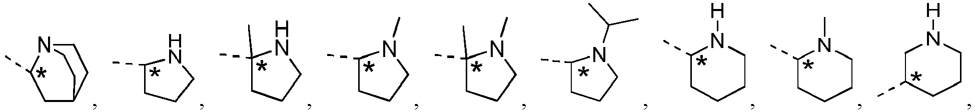 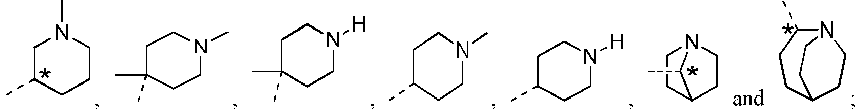 alternatively, the structural unit 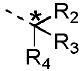 is selected from, further from 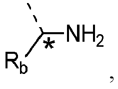 further more from

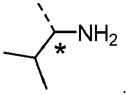

10. The compound, the isomer thereof or the pharmaceutically acceptable salt thereof according to claim 1, wherein,
the carbon atom with "^{∗}" can be a chiral carbon atom present in a form of a single (*R*) or (*S*) enantiomer or in a form rich in one enantiomer;
L is selected from -CH₂-CH₂-CH₂-;
R₁ is selected from H;
R₂ is selected from R_{b}, R₃ is selected from NH₂, and R₄ is selected from H;
alternatively, R₂ is selected from R_{c}, and R₃ and R₄ are joined to form a ring A group selected from 4-14 membered heterocycloalkyl each independently containing 1, 2 or 3 heteroatoms or heteroatom groups independently selected from the group consisting of N and NR_{d};
R_{b} is selected from C₁₋₆ alkyl;
R_{c} is selected from the group consisting of H and C₁₋₃ alkyl;
R_{d} is selected from the group consisting of H and C₁₋₄ alkyl.

11. The compound, the isomer thereof or the pharmaceutically acceptable salt thereof according to claim 1, wherein the compound is selected from the group consisting of a compound of formula (I-1) and a compound of formula (1-2), an isomer thereof and a pharmaceutically acceptable salt thereof, 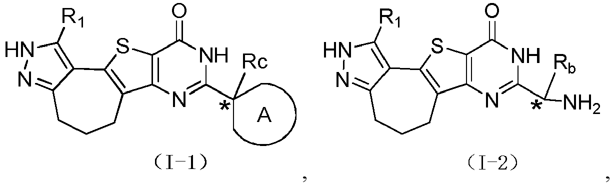 wherein the carbon atom with "^{∗}" can be a chiral carbon atom present in a form of a single (*R*) or (*S*) enantiomer or in a form rich in one enantiomer; R₁ is as defined in claim 1; the ring A group is as defined in claim 1; R_{b} is as defined in claim 1.

12. The following compounds, an isomer thereof or a pharmaceutically acceptable salt thereof,

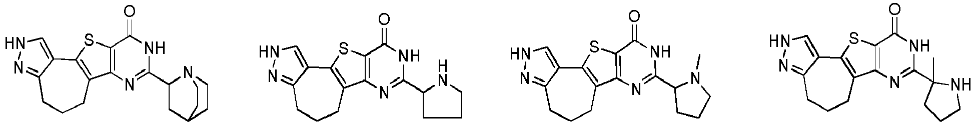

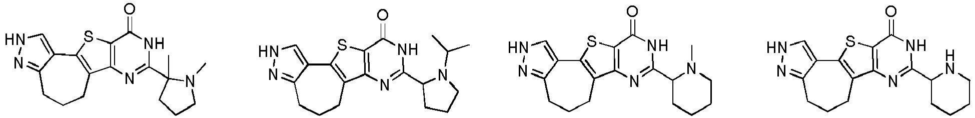

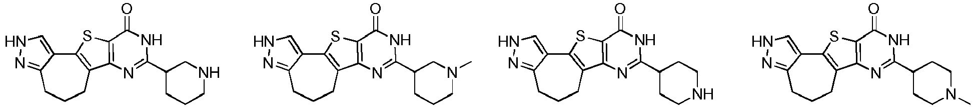

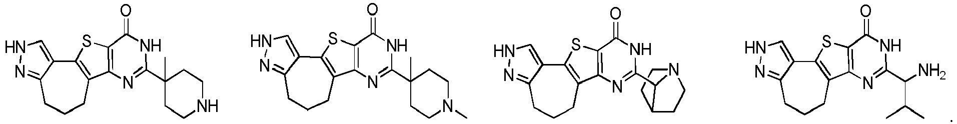

13. The compound, the isomer thereof or the pharmaceutically acceptable salt thereof according to claim 12, selected from the group consisting of

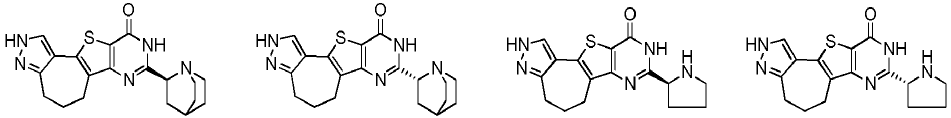

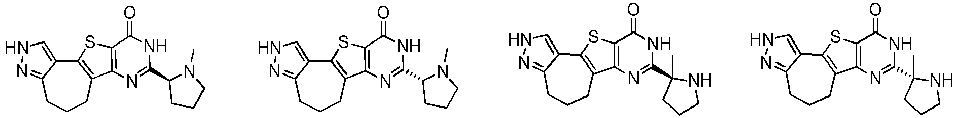

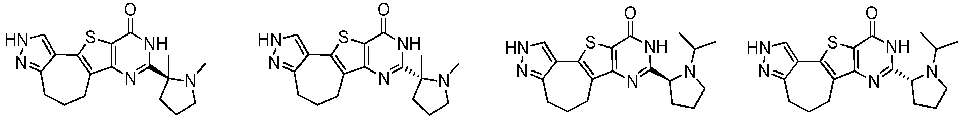

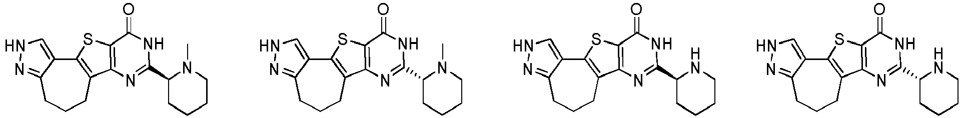

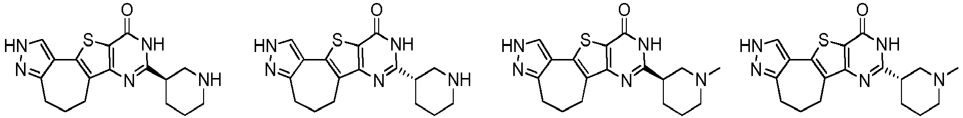

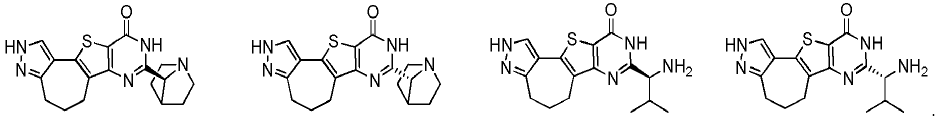

14. A pharmaceutical composition comprising a therapeutically effective amount of the compound or the pharmaceutically acceptable salt thereof according to any one of claims 1-13 and a pharmaceutically acceptable carrier.

15. The compound or the pharmaceutically acceptable salt thereof according to any one of claims 1-13 or the pharmaceutical composition according to claim 14 for use in treating a Cdc7 kinase-mediated disease, wherein optionally, the Cdc7 kinase-mediated disease is selected from a tumor; optionally the Cdc7 kinase-mediated disease is selected from colorectal cancer and pancreatic cancer.
